# EUROPÄISCHE PATENTANMELDUNG

(11) **EP 1 048 732 A1**
(43) Veröffentlichungstag der Anmeldung: **02.11.2000**
(21) Anmeldenummer: 99107412.1
(22) Anmeldetag: 26.04.1999
(51) Int. Cl.: C12N 15/58, C12N 15/62, C12N 15/31, C12N 9/72, C07K 14/245, C07K 1/113, C12N 1/20, C12N 15/70, C12P 21/02

(54) **Verfahren zur Herstellung von natürlich gefalteten und sekretierten Proteinen**

(71) Anmelder: F. HOFFMANN-LA ROCHE AG, 4070 Basel (CH)
(72) Erfinder: Die Erfindernennung liegt noch nicht vor
(74) Vertreter: Schreiner, Siegfried, Dr.

(57) **Zusammenfassung**

Ein Verfahren zur Herstellung eines wasserlöslichen, natürlich gefalteten eukaryontischen Polypeptids, enthaltend zwei oder mehrere über Disulfidbrücken verknüpfte Cysteine, durch Kultivierung prokaryontischer Zellen, a) wobei die genannten prokaryontischen Zellen einen Expressionsvektor enthalten, der für das genannte Polypeptid, das am N-Terminus eine prokaryontische Signalsequenz enthält, codiert, b) unter Bedingungen, bei denen das Polypeptid in das Periplasma oder das Medium sekretiert wird, c) Abspaltung der Signalsequenz und Isolierung des Polypeptids aus dem Periplasma oder dem Medium, dadurch gekennzeichnet, daß die Kultivierung in Gegenwart von Arginin oder einer Verbindung der allgemeinen Formel I R₂-CO-NRR₁ (I) erfolgt, wobei R und R₁ Wasserstoff oder eine gesättigte oder ungesättigte verzweigte oder unverzweigte C₁ - C₄-Alkylkette und R₂ Wasserstoff, NHR₁ oder eine gesättigte oder ungesättigte verzweigte oder unverzweigte C₁ - C₃-Alkylkette darstellen, ist zur rekombinanten Herstellung von Polypeptiden in Prokaryonten mit hoher Ausbeute geeignet.

## Beschreibung

Die Erfindung betrifft ein Verfahren zur Herstellung von wasserlöslichen, natürlich gefalteten und sekretrierten Polypeptiden nach Expression in prokaryontischen Zellen.

In prokaryontischen Organismen findet die Proteinsynthese, auch Translation genannt, an den Ribosomen im Cytoplasma statt. Bei einer Expression rekombinanter DNA in prokaryontischen Wirtsorganismen ist es oft wünschenswert, daß das dabei erhaltene rekombinante Genprodukt bzw. Protein aus dem Cytoplasma durch die innere bakterielle Membran in den periplasmatischen Raum zwischen innerer und äußerer Membran sekretiert wird. Vom Periplasma können sekretierte Proteine dann z.B. durch einen osmotischen Schock in das Nährmedium freigesetzt werden. Ein Nachteil dieses Verfahrens ist, daß die sezernierten Polypeptide häufig nicht die native, biologisch aktive Konformation ausbilden (Hockney, TIBTECH 12 (1994) 456 - 463).

In jüngster Zeit wurden molekulare Chaperone und Faltungskatalysatoren, wie Peptidyl-Prolyl-cis/trans-Isomerasen oder Proteindisulfidisomerasen (Glockshuber et al., EP-A 0 510 658) eingesetzt, um die Ausbeute an nativem rekombinanten Protein bei der Faltung in vivo zu erhöhen (Thomas et al., Appl. Biochem. Biotechnol. 66 (1997) 197-238). Dies führte teilweise zu erheblichen Verbesserungen bei der Expression z.B. von Ribulosebisphosphat-Carboxylase (RUBISCO; Goloubinoff et al., Nature 337 (1989) 44-47), humaner Procollagenase (Lee & Olins, J. Biol. Chem. 267 (1992) 2849-2852) oder neuronaler Stickstoffoxidsynthase aus Ratten (Roman et al., Proc. Natl. Acad. Sci. USA 92 (1995) 8428-8432). In diesen Beispielen wurden GroEL/ES bzw. das DnaK-System aus E. coli im Cytosol co-überexprimiert. Der positive Effekt liegt dabei meist in einer erhöhten Anreicherung des gewünschten Proteins in löslicher Form.

Auch bei der Sekretion rekombinanter Proteine ins Periplasma von E. coli wurde die Co-Expression von Chaperone untersucht. Hier wurde jedoch nur eine cytosolische Überexpression von Chaperone erprobt, um die Sekretion ins Periplasma zu optimieren (Perez-Perez et al., Biochem. Biophys. Res. Commun. 210 (1995) 524-529; Sato et al., Biochem. Biophys. Res. Commun. 202 (1994) 258-264; Berges et al., Appl. Environ. Microbiol. 62 (1996) 55-60). Bisherige Versuche zur Co-Sekretion in E. coli betrafen nur Faltungshelfer-Proteine, wie z. B. Proteindisulfidisomerase (PDI; Glockshuber et al., EP-A 0 510 658) oder Peptidyl-Prolyl-cis/trans-Isomerasen oder Dsb-Proteine (Knappik et al., Bio/Technology 11(1993) 77-83; Qiu et al., Appl. Environm. Microbiol. 64 (1998) 4891-4896 und Schmidt et al., Prot. Engin. 11 (1998) 601 - 607.

Verbindungen wie Harnstoff oder Harnstoffderivate, Formamid, Acetamid oder L-Arginin werden bei Verfahren zur in vitro Renaturierung von unlöslichen Proteinaggregaten (Inclusion bodies - Einschlußkörper) eingesetzt, die bei der cytoplasmatischen Expression von rekombinanter DNA in prokaryontischen Zellen entstehen. L-Arginin kann als Zusatz bei der Renaturierung in vitro die Ausbeute an nativ gefalteten Proteinen erheblich verbessern (Rudolph et al., US-Patent Nr. 5,593,865; Buchner & Rudolph, Bio/Technology 9 (1991)157-162; Brinkmann et al., Proc. Natl. Acad. Sci USA 89 (1992) 3075-3079; Lin & Traugh, Prot. Express. Purif. 4 (1993) 256-264).

Aufgabe der Erfindung ist es, ein Verfahren zur Herstellung von wasserlöslichen, natürlich gefalteten eukaryontischen Polypeptiden nach Expression in Prokaryonten zur Verfügung zu stellen, welches auf einfache Weise durchführbar ist und bei dem eine aufwendige in vitro-Nachbehandlung, wie Auflösung von inclusion bodies, Reduktion und Naturierung, nicht erforderlich ist.

Die Aufgabe wird gelöst durch ein Verfahren zur Herstellung eines wasserlöslichen, natürlich gefalteten eukaryontischen Polypeptids, enthaltend zwei oder mehrere über Disulfidbrücken verknüpfte Cysteine, durch Kultivierung prokaryontischer Zellen,
a) wobei die genannten prokaryontischen Zellen einen Expressionsvektor enthalten, der für das genannte Polypeptid, das am N-Terminus eine prokaryontische Signalsequenz enthält, codiert
b) unter Bedingungen, bei denen das Polypeptid in das Periplasma oder das Medium sekretiert wird,
c) Abspaltung der Signalsequenz und Isolierung des Polypeptids aus dem Periplasma oder dem Medium,
dadurch gekennzeichnet, daß die Kultivierung in Gegenwart von Arginin oder einer Verbindung der allgemeinen Formel I

R₂-CO-NRR₁ (I)

erfolgt, wobei
R und R₁ Wasserstoff oder eine gesättigte oder ungesättigte verzweigte oder unverzweigte C₁-C₄-Alkylkette und
R₂ Wasserstoff, NHR₁ oder eine gesättigte oder ungesättigte verzweigte oder unverzweigte C₁-C₃-Alkylkette darstellen.

Vorzugsweise beträgt die Konzentration von Arginin oder der Verbindung der allgemeinen Formel I mindestens 0,1 mol/l, kann aber auch deutlich höher sein, solange die Löslichkeit von Arginin oder der genannten Verbindung gewährleistet ist. Bevorzugt werden Arginin oder die Verbindungen der allgemeinen Formel I in einer Konzentration von 0,1 bis 1,5 mol/l verwendet.

Als Verbindungen der allgemeinen Formel I werden vorzugsweise Formamid, Acetamid, Harnstoff oder Harnstoffderivate, wie Ethylharnstoff oder Methylharnstoff zum Nährmedium, welches für die Kultivierung der prokaryontischen Zellen verwendet wird, zugesetzt. Arginin kann beispielsweise als Hydrochlorid oder als andere titrierte Form der Base Arginin verwendet werden. Vorzugsweise wird jedoch L-Arginin, besonders bevorzugt die Hydrochloridform von L-Arginin, verwendet.

In einer bevorzugten Ausführungsform des erfindungsgemäßen Verfahrens werden dem zur Kultivierung der prokaryontischen Zellen verwendeten Nährmedium (Fermentationsmedium) zusätzlich reduzierende Thiolreagenzien, welche SH-Gruppen enthalten, zugesetzt, wobei die Ausbeute an rekombinant gewonnenem Protein weiter erhöht wird. Vorzugsweise werden 0,1-15 mmol/l Thiolreagenz zugesetzt. Erfindungsgemäß ist unter dem Begriff "Thiolreagenz" entweder ein reduzierendes (reduziertes) Thiolreagenz mit SH-Gruppen oder ein Gemisch von reduzierenden Thiolreagenzien mit SH-Gruppen und oxidierenden Thiolreagenzien mit Disulfidgruppen zu verstehen. Bevorzugte Substanzen sind reduziertes Glutathion (GSH), Cystein, N-Acetylcystein, Cysteamin, β-Mercaptoethanol und ähnliche Verbindungen. Die Thiolreagenzien können sowohl einzeln als auch in Gemischen verwendet werden. Besonders geeignet sind Thiolreagenzien wie beispielsweise Glutathion (GSH), die eine einzige SH-Gruppe pro Molekül aufweisen. Thiolreagenzien wie Glutathion, sind für die Verbesserung der Ausbeute nativ gefalteter Proteine bei der Expression rekombinanter DNA in prokaryontischen Zellen bekannt (Glockshuber et al., EP-A 0 510 658).

In einer weiteren bevorzugten Ausführungsform des erfindungsgemäßen Verfahrens werden zusätzlich molekulare Chaperone überexprimiert und cosekretiert. Unter Chaperonen gemäß der Erfindung sind Proteine zu verstehen, die andere, nicht-native Proteine in vivo vor Aggregation schützen und die Ausbildung ihrer nativen Konformation fördern. Molekulare Chaperone werden im Stand der Technik eingesetzt, um Proteine zu stabilisieren und damit vor Aggregation und Inaktivierung zu schützen (Buchner et al., EP-A 0 556 726 A1). Vorzugsweise werden ATP-unabhängige Chaperone des HSP40-Typs (Molmasse ca. 40 kDa) oder ein kleines Hitzeschockprotein (sHSP) verwendet. DnaJ ist ein 40 kDa Hitzeschockprotein, das im Cytoplasma von E. coli vorkommt und Teil des sogenannten Hsp70-Chaperonsystems ist (Bukau, B. & Horwich, A., Cell 92 (1998) 351-366). Zu diesem System gehören außerdem DnaK (Hsp70) und GrpE. Bestimmte Proteine werden durch das DnaK-System in einem ATP-abhängigen Prozeß zur nativen Konformation gefaltet (Schröder et al., EMBO J. 12 (1993) 4137-4144; Langer et al., Nature 356 (1992) 683 - 689). Zur Rückfaltung denaturierter Proteine benötigt dieses System zusätzlich ATP. DnaJ schützt in Abwesenheit von DnaK und ATP nicht-native Proteine vor Aggregation und vermittelt einen Faltungs-kompetenten Zustand (Schröder et al., EMBO J. 12 (1993) 4137-4144). Weiterhin bevorzugt ist die Co-Sekretion eines N-terminalen Fragmentes von DnaJ, das die Aminosäuren 1-108 umfaßt und im Folgenden als "J-Domäne" (Kelley, TIBS 23 (1998) 222-227) bezeichnet wird. In diesem Bereich befinden sich die J-Domäne und eine G/F-reiche Domäne, die Wechselwirkungen mit DnaK ausüben (Wall et al., J. Biol. Chem. 270 (1995) 2139-2144). Es wurde gezeigt, daß die Co-Expression von DnaJ im Cytosol zur Erhöhung der Ausbeute an löslichem Protein führen kann (Yokoyama et al., Microbiol. Ferment. Technol. 62 (1998) 1205-1210).

Hsp25 (z.B. aus der Maus) ist ein Vertreter der kleinen Hitzeschockproteine (Gaestel et al., Eur. J. Biochem. 179 (1989) 209-213), einer Klasse von Chaperonen, die ubiquitär verbreitet ist. Die Molmasse dieser Proteine liegt zwischen 15 und 30 kDa. Bei Hitzeschock werden die sHsps in der Zelle stark angereichert (bis zu 1% des Gesamtzellproteins - Arrigo & Landry (1994), In Morimoto (Hrsg.): The Biology of Heat Shock Proteins and Molecular Chaperones, Cold Spring Harbour Press, 335-373). Wie DnaJ-Proteine besitzen sHsps die Eigenschaft, die Aggregation von nichtnativen Proteinen zu verhindern und diese in einem faltungskompetenten Zustand zu halten (Jakob et al., J. Biol. Chem. 268 (1993) 1517-1520; Ehrsperger et al., EMBO J. 16 (1997) 221-229).

Der Begriff "Überexpression" gemäß vorliegender Erfindung bedeutet eine Steigerung der Expression der sekretierten Proteine wie z.B. DnaJ- und Hsp25 (vorzugsweise um mindestens 100%) im Vergleich zur Expression im Wildtyp des jeweils verwendeten prokaryontischen Wirtsorganismus. Eine solche Überexpression läßt sich z.B. dadurch erreichen, daß sich die Gene (für das Protein, Chaperon und/oder Signalpeptid) unter Kontrolle eines starken prokaryontischen, vorzugsweise induzierbaren Expressionssignals (z.B. eines lac- oder T7-Promotors oder eines Derivates davon) befinden.

Das Sekretionskonstrukt für die Überexpression der Polypeptide (Proteine) samt regulatorischer Regionen (Promotor und Terminator) auf der rekombinanten DNA ist vorzugsweise in einen Vektor, welcher zusätzlich die in Prokaryonten seltene Arginin-tRNA _{AGA/AGG} codiert integriert oder wird mit einem Vektor, welcher diese tRNA codiert, co-exprimiert (Brinkmann et al., Gene 85 (1989) 109-114). Dies ermöglicht sowohl die Co-Überexpression der jeweiligen Proteine ins bakterielle Periplasma als auch die Transkription der seltenen tRNA^{Arg}_{AGA/AGG}, was eine erhöhte Synthese des gewünschten Proteins im bakteriellen Wirtsorganismus zur Folge hat.

Unter einer prokaryontischen Signalsequenz im Sinne der Erfindung ist ein Nukleinsäurefragment zu verstehen, welches aus Prokaryonten, vorzugsweise aus gramnegativen Bakterien, abgeleitet ist und das Durchdringen von an das Signalpeptid gebundenen Proteinen durch die inneren bakteriellen Membranen gewährleistet. Dadurch werden die Proteine im Periplasma bzw. im Zellüberstand lokalisiert. Solche Signalsequenzen haben üblicherweise eine Länge von 18 - 30 Aminosäuren und sind beispielsweise beschrieben in Murphy & Beckwith: Export of Proteins to the Cell Envelope in Escherichia coli und in Neidhardt et al. (Hrsg.): Escherichia coli and Salmonella, Second Edition, Vol. 1, ASM Press, Washington, 1996, S. 967-978. Die Abspaltung von bakteriellen Signalsequenzen kann z.B. nach einer Sequenz Ala-X-Ala stattfinden (von Heijne et al., J. Mol. Biol. 184 (1985) 99-105). Die Struktur der bakteriellen Signalpeptidase ist beschrieben in Paetzel et al., Nature 396 (1998) 186-190. Vorzugweise werden Signalsequenzen verwendet, welche durch im Periplasma von prokaryontischen Zellen lokalisierten Proteasen vom gewünschten Protein wieder abgespalten werden. Alternativ kann durch Zugabe solcher Proteasen zum Zellüberstand oder zum isolierten Protein die Abspaltung der Signalsequenz erfolgen.

Mit dem erfindungsgemäßen Verfahren kann die heterologe Expression einer Vielzahl von eukaryontischen Proteinen wie z.B. Proteasen, Interferone, Proteinhormone, Antikörper oder Fragmenten davon verbessert werden. Besonders geeignet ist das Verfahren für die heterologe Herstellung von Proteinen, die im nativen Zustand mindestens zwei über eine Disulfidbrücke verknüpfte Cysteine enthalten und dann, wenn sie N-terminal keine fusionierte prokaryontische Signalsequenz besitzen, bei der prokaryontischen Expression als unlösliche inclusion bodies entstehen. Besonders geeignet ist das Verfahren für Proteine, die mehr als 5 Disulfidbrücken in nativem Zustand enthalten. Ein solches Protein ist beispielsweise ein rekombinanter Plasminogenaktivator (im folgenden rPA genannt, Martin et al., Cardiovasc. Drug Rev. 11 (1993) 299-311, US-Patent Nr. 5,223,256). rPA besitzt 9 Disulfidbrücken, die im reduzierenden Cytosol von E. coli nicht ausgebildet werden.

Dabei wird die periplasmatische Lokalisation des Proteins, und gegebenenfalls des Chaperons, durch "operative Verknüpfung" mit einem Signalpeptid zum Durchdringen innerer bakterieller Membranen gewährleistet.

Bei der Expression eines solchen Plasminogenaktivators stellte sich eine Konzentration von 0,4 mol/l L-Arginin und 5 mmol/l Glutathion (bei Co-Sekretion von DnaJ, J-Domäne, Hsp25 sowie ScFv) bzw. 0,4 mol/l L-Arginin ohne Glutathion (ohne Co-Sekretion von DnaJ) als optimal heraus.

Zur Gewinnung des sekretorischen rPA-Proteins in funktionaler Form in E. coli wurde das Gen für dieses Protein aus dem Plasmid pA27fd7 (Kohnert et al., Protein Engineering 5 (1992) 93-100) mit gentechnologischen Mitteln an eine prokaryontische Signalsequenz gramnegativer Bakterien, beispielsweise an die Signalsequenz von Pectatlyase B (PelB) von Erwinia amylovora, fusioniert. Die Genfusion wurde durch Klonierung in den Vektor pET20b(+) (Novagen Inc., Madison, USA) hergestellt. Damit unterliegt die Genexpression der Kontrolle des T7-Promotors. Die im Fusionsprotein vorhandene Signalsequenz bewirkt die Sekretion ins Periplasma. Während oder nach der Sekretion wird die Signalsequenz durch eine in der inneren Membran lokalisierte Peptidase abgespalten. Sezerniertes Protein kann dann im Periplasma falten. Die oxidierenden Bedingungen dieses Kompartiments ermöglichen die Ausbildung von Disulfidbrücken. Durch den erfindungsgemäßen Zusatz von niedermolekularen faltungsverbessernden Proteinen und Thiolragenzien im Nährmedium und gleichzeitiger Co-Überexpression von DnaJ, J-Domäne oder Hsp25 im Periplasma gelingt es, die Ausbeute an funktionalen Protein um mehr als das 100-fache zu steigern.

Weitere Beispiele von erfindungsgemäßen Polypeptiden sind Antikörper oder Antikörperfragmente, beispielsweise ein Single-Chain F_{V}-Fragment (ScFᵥ, z.B. gegen das Schilddrüsenstimulierende Hormon (thyroide stimulating hormone, TSH). ScFvs sind verkürzte Antikörper, die nur aus den variablen Abschnitten (F_{V}) der schweren und leichten Kette eines Antikörpers bestehen, die über einen kurzen (meist Gly₄Ser₃) Linker künstlich fusioniert sind (Hudson, Curr. Opin Biotechnol. 9 (1998) 395-402). ScF_{V}s haben normalerweise die gleiche Affinität zum Antigen wie die paternalen Fv-Stränge, können jedoch in E. coli überexprimiert werden. Da sie stabilisierende Intradomänen-Disulfidbrücken besitzen, die essentiell für die Stabilität sind, führt eine Expression im Cytosol meist zur Bildung von Inclusion Bodies (Übersichtsartikel: Shibui et al., Appl. Microbiol. Biotechnol. 37 (1992) 352-357). ScF_{V}s können durch Zufallsmutationen und anschließende Phage-Display-Selektion auf Bindung gewünschter Antigene gezielt optimiert werden (Übersichtsartikel: Allen et al., TIBS 20 (1995) 511-516). Durch Zugabe von 5 mM GSH und 0,4 M L-Arginin konnte die Ausbeute an funktionellem ScFv-TSH im Periplasma um das 10-fache und im Medienüberstand um das 40-fache gegenüber einer Kultivierung ohne Zusätze verbessert werden.

Die folgenden Beispiele, Publikationen, das Sequenzprotokoll und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beschreibung des Sequenzprotokolls

SEQ ID NO: 1 und 2 zeigen die Sequenz des Teils des Expressionsplasmides pUBS520-pIN-dnaJ, der für das Fusionsprotein aus OmpA-Signalsequenz und DnaJ codiert, zusammen mit den regulatorischen Sequenzen (Promotor, Terminator), die aus pIN III ompA3-dnaJ amplifiziert wurden.

SEQ ID NO: 3 und 4 zeigen die Sequenz des Teils des Expressionsplasmides pUBS520-pIN-J-Domain, der für das Fusionsprotein aus OmpA-Signalsequenz und J-Domäne codiert, zusammen mit den regulatorischen Sequenzen (Promotor, Terminator), die aus pIN III ompA3-dnaJ amplifiziert wurden.

SEQ ID NO: 5 und 6 zeigen die Sequenz des Teils des Expressionsplasmides pUBS520-pIN-hsp25, der für das Fusionsprotein aus OmpA-Signalsequenz und Hsp25 codiert, zusammen mit den regulatorischen Sequenzen (Promotor, Terminator), die aus pIN III ompA3-hsp25 amplifiziert wurden.

SEQ ID NO: 7 und 8 zeigen die Sequenz des Teils des Expressionsplasmides pUBS520-ScFvOx, der für das Fusionsprotein aus PelB-Signalsequenz und ScFvOxazolon codiert, zusammen mit den regulatorischen Sequenzen (Promotor, Terminator), die aus pHEN-ScFv bzw. pIN III ompA3 amplifiziert wurden.

SEQ ID NO: 9 und 10 zeigen die Sequenz des Teils des Expressionsplasmides pET20b(+)-rPA, der für das Fusionsprotein aus PelB-Signalsequenz und rPA codiert.

### Beschreibung der Figuren

**Fig. 1** zeigt die Abhängigkeit der Expression von nativem rPA im Periplasma von *E. coli* bei 5 mM GSH in Abhängigkeit von der L-Argininkonzentration und verschiedener Co-Sekretionskonstrukte.

**Fig. 2** zeigt einen Vergleich der Expression von rPA im Periplasma von *E. coli* BL21(DE3) bei Co-Sekretion von DnaJ und bei Zusatz von GSH und verschiedenen niedermolekularen faltungsverbessernden Stoffen zum Medium.

**Fig. 3** zeigt eine schematische Darstellung des Expressionsplasmides pUBS520-pIN-dnaJ.

**Fig. 4** zeigt eine schematische Darstellung des Expressionsplasmides pUBS520-pIN-J-Domain.

**Fig. 5** zeigt eine schematische Darstellung des Expressionsplasmides pUBS520-pIN-hsp25.

**Fig. 6** zeigt eine schematische Darstellung des Expressionsplasmides pUBS520-ScFvOx.

**Fig. 7** zeigt eine schematische Darstellung des Expressionsplasmides pET20b(+)-rPA.

**Fig. 8** zeigt die Abhängigkeit der Expression von funktionellem ScFv-TSH von der Konzentration von L-Arginin und GSH im Kulturmedium.

(Werte wurden als relative Mengen bezüglich der Probe ohne Medienzusätze (:1) berechnet.)

**Fig. 9** zeigt die Abhängigkeit der Expression von funktionellem ScFv-TSH von der Konzentration von L-Arginin und GSH im Periplasma von *E. coli.*

(Werte wurden als relative Mengen bezüglich der Probe ohne Medienzusätze (:1) berechnet.)

### Allgemeines:

Zur periplasmatischen Überexpression von DnaJ, der J-Domäne sowie Hsp25 in E. coli wurde die DNA, die für diese Proteine codiert, mit gentechnologischen Mitteln an die Signalsequenz des Outer Membrane Proteins A (OmpA) von E. coli fusioniert und die Fusion auf einem rekombinanten Plasmid unter Kontrolle des lac-lpp-Promotors in E. coli exprimiert. Somit werden die Polypeptidkette von DnaJ und Hsp25 ins Periplasma des prokaryontischen Wirtsorganismus transportiert und dort nativ gefaltet. Die Lokalisation und native Faltung konnte dabei durch limitierte Proteolyse mit Trypsin und Western Blot nachgewiesen werden.

### Beispiel 1:

### Konstruktion des Expressionsplasmides pIN III omp A3-dnaJ

Molekulargenetische Techniken beruhten auf Ausubel et al. (Hrsg.), J. Wiley & Sons, 1997, Curr. Protocols of Molecular Biology. Oligonucleotide wurden von den Firmen MWG Biotech, Ebersberg oder GIBCO Life Sciences, Eggenstein, DE bezogen.

Das Gen, das für DnaJ codiert, Gene Bank Accession No. M 12565, wurde über die Restriktionsschnittstellen EcoRI und BamHI in das Expressionsplasmid pIN III ompA3 (Ghayreb et al., EMBO J. 3 (1984) 2437-2442) kloniert. Die Sequenz des klonierten PCR-Fragments wurde durch Didesoxy-Sequenzierung (LiCor DNA-Sequencer 4000, MWG Biotech, Ebersberg) überprüft. Das resultierende Plasmid wurde pIN III ompA3-dnaJ bezeichnet. Die Sequenz des periplasmatisch exprimierten DnaJ unterscheidet sich von dem Wildtyp-Protein dahingehend, daß die Polypeptidsequenz mit Gly-Ile-Pro beginnt statt mit Met, es fand somit eine Verlängerung des N-Terminus um 2 Aminosäuren statt. DnaJ befindet sich damit unter Kontrolle des lac-lpp-Promotors, der mit IPTG (Isopropyl-β-D-Thiogalactosid) induziert wird.

### Beispiel 2:

### Konstruktion des Expressionsplasmides pUBS520-pIN-dnaJ

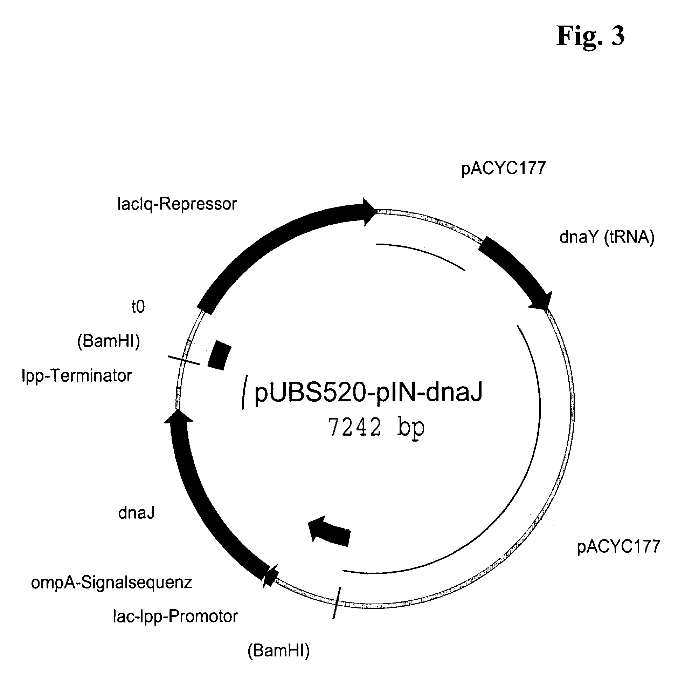

Mittels PCR wurde der Bereich aus dem Plasmid pIN III ompA3-dnaJ amplifiziert, der für das lac-lpp Operon, die Signalsequenz, das dnaJ-Gen und die Terminator-Region des Operons codiert (SEQ ID NO: 1). Das PCR-Produkt wurde mit der Restriktionsendonuclease BglII geschnitten und in den mit der Restriktionsendonuclease BamHI linearisierten Vektor pUBS520 kloniert. Das resultierende Plasmid wurde pUBS520-pIN-dnaJ bezeichnet (Fig. 3).

### Beispiel 3:

### Konstruktion des Expressionsplasmides pUBS 520-pIN-J-Domain

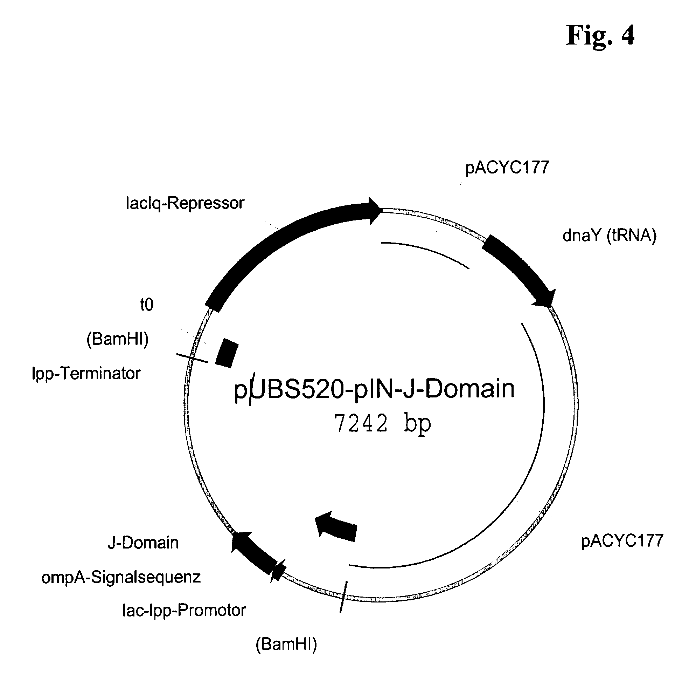

Mittels des QuikChange-Mutagenese-Systems (Promega, Mannheim, DE) wurden im Plasmid pUBS 520-pIN-dnaJ nach dem Nucleotid 324 zwei Stop-Codone eingefügt, so daß nur noch die ersten 108 Aminosäuren exprimiert werden. Die Sequenz des mutagenisierten Bereiches wurde durch Didesoxy-Sequenzierung (LiCor DNA-Sequencer 4000, MWG Biotech, Ebersberg) und die Expression des verkürzten Proteinfragments durch Western Blotting und Detektion mit einem Anti-DnaJ-Antikörper nachgewiesen. Das entstandene Plasmid wurde mit pUBS 520-pIN-J-Domain (Fig. 4) bezeichnet.

### Beispiel 4:

### Konstruktion des Expressionsplasmides pIN III ompA3-hsp25

Das Gen, das für Hsp25 codiert, Gene Bank Accession No.: L 07577, wurde über die Restriktionsschnittstellen EcoRI und BamHI in das Expressionsplasmid pIN III ompA3 (Ghayreb *et al.,* EMBO J. 3 (1984) 2437-2442) kloniert. Die Sequenz des klonierten PCR-Fragments wurde durch Didesoxy-Sequenzierung (LiCor DNA-Sequencer 4000, MWG Biotech, Ebersberg) überprüft. Das resultierende Plasmid wurde pIN III ompA3-hsp25 bezeichnet. Die Sequenz des periplasmatisch exprimierten Hsp25 unterscheidet sich von dem Wildtyp-Protein dahingehend, daß die Polypeptidsequenz mit Gly-Ile-Leu beginnt statt mit Met, es fand somit eine Verlängerung des N-Terminus um 2 Aminosduren statt. Hsp25 befindet sich damit unter Kontrolle des lac-lpp-Promotors, der mit IPTG (Isopropyl-β-D-Thiogalactosid) induziert wird.

### Beispiel 5:

### Konstruktion des Expressionsplasmides pUBS520-pIN-hsp25

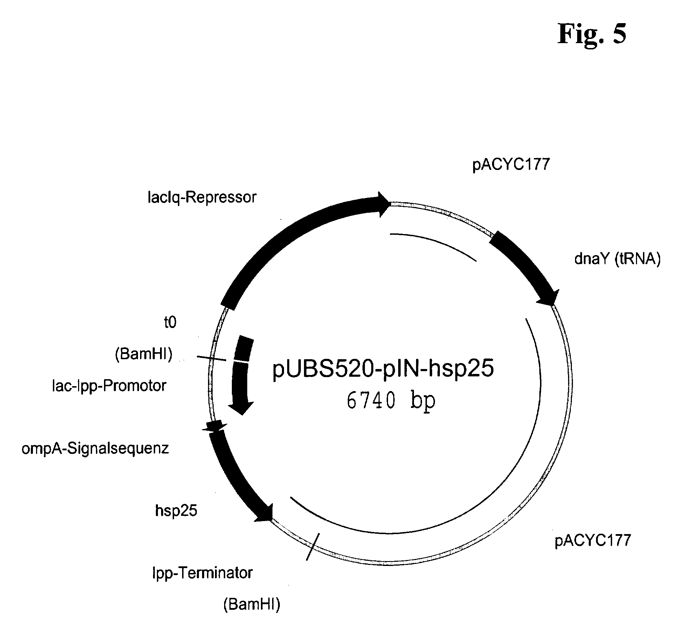

Mittels PCR wurde der Bereich aus dem Plasmid pIN III ompA3-hsp25 amplifiziert, der für das lac-lpp Operon, die Signalsequenz, das hsp25-Gen und die Terminator-Region des Operons codiert (SEQ ID NO: 5). Das PCR-Produkt wurde mit der Restriktionsendonuclease BglII geschnitten und in den mit der Restriktionsendonuclease BamHI linearisierten Vektor pUBS520 kloniert. Das resultierende Plasmid wurde pUBS520-pIN-hsp25 bezeichnet (Fig. 5).

### Beispiel 6:

### Konstruktion des Expressionsplasmides pUBS520-ScFvOx

Als negativ-Kontrolle wurde die Co-Expression eines Single-Chain-Fv-Fragmentes, das gegen das Hapten Oxazolon gerichtet ist (ScFvOxazolon; Fiedler und Conrad, Bio/Technology 13 (1995) 1090-1093 untersucht, das keine Chaperon-Eigenschaften besitzt.

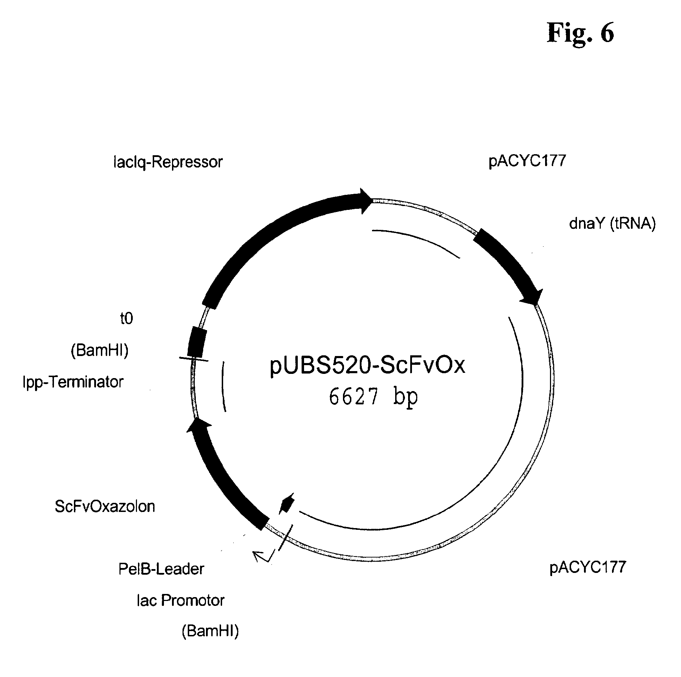

Mittels PCR wurde der Bereich aus dem Plasmid pHEN-ScFvOx amplifiziert, der für den lac-Promotor, die Signalsequenz pelB und das scfvox-Gen codiert. In einer zweiten PCR wurde der Bereich aus dem Plasmid pIN III ompA3 amplifiziert, der für den lpp-Terminator codiert. In einer anschließenden PCR wurden die beiden Fragmente fusioniert. Das so entstandene PCR-Produkt (SEQ ID NO: 7) wurde mit der Restriktionsendonuclease BglII geschnitten und in den mit der Restriktionsendonuclease BamHI linearisierten Vektor pUBS520 kloniert. Das resultierende Plasmid wurde pUBS520-ScFvOx bezeichnet (Fig. 6).

### Beispiel 7:

### Konstruktion des Expressionsplasmides pET20b(+)-rPA

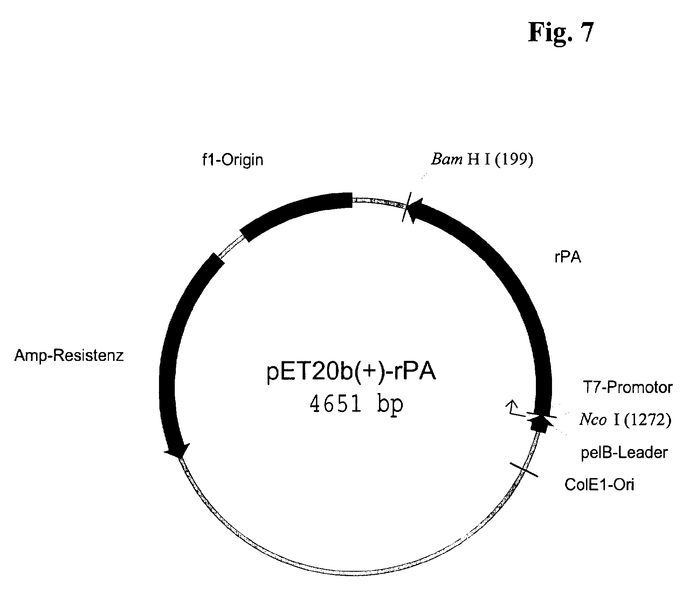

Mit Hilfe der PCR-Methode wurde das Gen eines Plasminogenaktivators (rPA) aus dem Plasmidvektor pA27fd7 (Kohnert et al., Protein Engineering 5 (1992) 93-100) amplifiziert. Das PCR-Produkt wurde mit den Restriktionsendonucleasen NcoI und BamHI gespalten und in den Plasmidvektor pET20b(+) (Novagen Inc., Madison, USA) kloniert. Das Plasmid codiert für ein Fusionsprotein, welches aus der Signalsequenz von PelB (Pectatlyase aus Erwinia amylovora) und rPA besteht und die Sekretion von rPA ins Periplasma Didesoxy-Sequenzierung (LiCor DNA-Sequencer 4000, MWG Biotech, Ebersberg, DE) überprüft. Das Konstrukt wurde als pET20b(+)-rPA bezeichnet (Fig. 7). In dem Plasmid wird rPA unter Kontrolle des T7-Promotors exprimiert, wobei die T7-RNA-Polymerase im Stamm E. coli BL21(DE3) der Kontrolle des lacUV5-Promotors unterliegt. Die Induktion erfolgt durch Zugabe von IPTG. Das periplasmatisch exprimierte rPA unterscheidet sich von dem bei Kohnert et al beschriebenen Plasminogenaktivator durch Austausch der zweiten Aminosäure (Ser) gegen Ala.

### Beispiel 8:

### Funktionale Expression von rPA im Periplasma von E. coli unter Verwendung der Medienzusätze Glutathion und L-Arginin

Eine stationäre Übernachtkultur von E. coli BL21(DE3) (Studier & Moffat, J. Mol. Biol. 189 (1986) 113-130), die mit pET20b(+)-rPA und pUBS520-pIN-dnaJ transformiert wurde (Co-Expression von DnaJ), eine Übernachtkultur von E. coli BL21(DE3), die mit pET20b(+)-rPA und pUBS520-pIN-J-Domain transformiert wurde (Co-Expression der J-Domäne), eine Übernachtkultur von E. coli BL21(DE3), die mit pET20b(+)-rPA und pUBS520-pIN-hsp25 transformiert wurde (Co-Expression von Hsp25), eine Übernachtkultur von E. coli BL21(DE3), die mit pET20b(+)-rPA und pUBS520-ScFvOx transformiert wurde (Co-Expression von ScFvOx), eine Übernachtkultur von E. coli BL21(DE3), die mit pET20b(+)-rPA und pUBS520 transformiert wurde bzw. eine Übernachtkultur von E. coli BL21(DE3), die mit pET20b(+) und pUBS520 transformiert wurde (Kontrollkultur), wurde im Verhältnis 1:50 in 100 ml LB-Medium mit Ampicillin (100 µg/ml) und Kanamycin (50 µg/ml, Fluka Chemica, Neu-Ulm, DE) verdünnt und bei 24°C und 170 rpm geschüttelt. Nach 3 h Wachstum wurden je 5ml der Kultur zu je 10 ml LB-Medium mit o.g. Mengen an Ampicillin und Kanamycin und verschiedenen Konzentrationen von GSH (0-10 mM, Fluka, DE) und L-Arginin HCl (0-0,4 M, ICN) gegeben und mit jeweils 1mM IPTG (Isopropyl-β-D-Thiogalactosid, AppliChem, Darmstadt, DE) induziert. Die Zellen wurden weitere 21 h bei 24 °C und 170 rpm geschüttelt und nach Bestimmung der OD₆₀₀ eine 1 ml-Probe genommen. Diese 1ml-Zellproben wurden nach einer modifizierten Vorschrift nach Jacobi et al. (J. Biol. Chem. 272 (1997) 21692-21699) in 2 ml-Eppendorf-Reaktionsgefäßen fraktioniert. Im Detail wurde das Zellpellet mit 500 µl Fraktionierungspuffer (150 mM NaCl (Roth GmbH), 50 mM Tris/HCl (Roth GmbH, 5mM EDTA (Biomol) und 1 mg/ml Polymyxin-B-Sulfat (Sigma), pH 7,5) versetzt, 1 h bei 10 °C auf einem Eppendorf-Thermoschüttler bei 1400 rpm geschüttelt und dann 15 min bei 14 000 rpm in einer auf 10°C gekühlten Eppendorf-Mikrozentrifuge zentrifugiert, so daß eine Fraktion mit den löslichen periplasmatischen Proteinen (Überstand) und eine Restfraktion (Pellet) entstand.

Die Bestimmung von der Aktivität von rPA erfolgte im wesentlichen nach der Methode von Verheijen et al. Thromb. Haemostasis 48 (1982) 266-269).

Alle ermittelten rPA-Konzentrationen in den Zellextrakten wurden auf Zellsuspensionen von OD₆₀₀=1 normiert, um den Fehler, der bei der Messung in verschiedenen Puffern auftritt, zu korrigieren.

### Beispiel 9:

### Funktionale Expression von rPA im Periplasma von E. coli unter Verwendung von Gemischen aus Glutathion mit Formamid, Methylformamid, Acetamid, Methylharnstoff sowie Ethylharnstoff als Medienzusätze

Eine stationäre Übernachtkultur von E. coli BL21(DE3), die mit pET20b(+)-rPA und pUBS520-pIN-dnaJ transformiert wurde (Co-Expression von DnaJ), wurde wie in Beispiel 8 angegeben kultiviert. Zusätzlich wurden Verbindungen der Formel I und jeweils 5 mM Glutathion dem Kulturmedium zugesetzt. Eine Kontrollkultur wurde in LB ohne Zusätze kultiviert. Die Verbindungen der Formel I und ihre eingesetzte Konzentration sind in Tabelle 3 aufgeführt. Die Probenaufbereitung, Periplasmafraktionierung und der Enzymtest auf tPA-Aktivität wurden wie in Beispiel 8 ausgeführt.

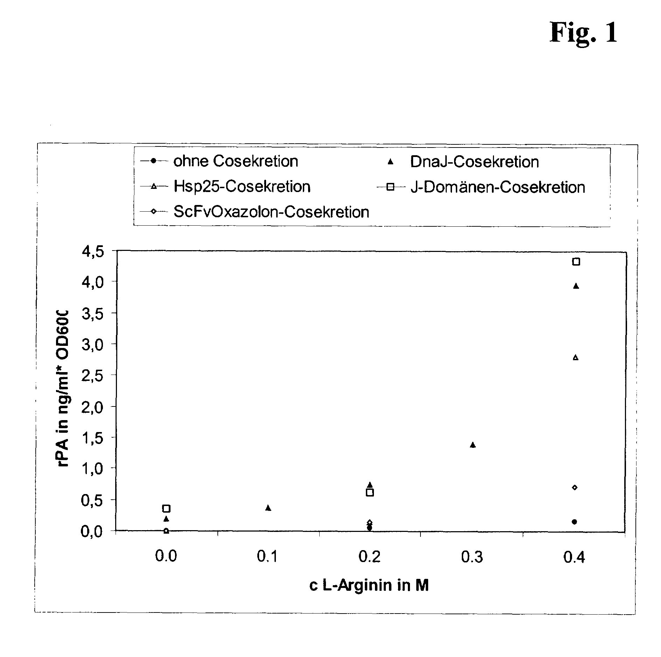

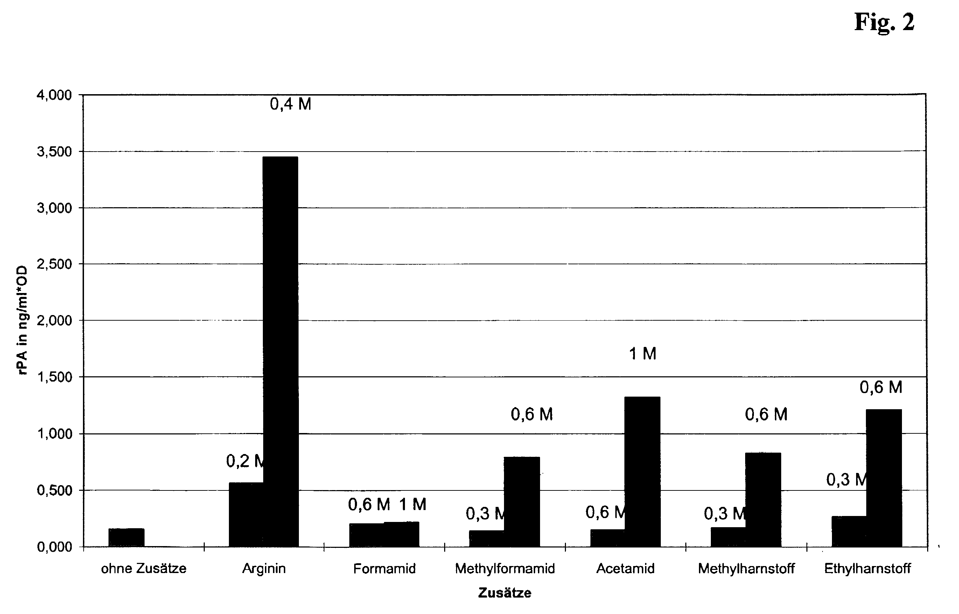

Die Ergebnisse zur rPA Expression zeigen die Tabellen 1 und 2 sowie die Figuren 1 und 2.

**Tabelle 1:**

| **Effekt von L-Arginin im Fermentationsmedium auf die Ausbildung von nativem rPA im Periplasma** | | | | | | |
|---|---|---|---|---|---|---|
| **Co-sekretiertes Protein** | **OM L-Arginin** | | **0,2 M L-Arginin** | | **0,4 M L-Arginin** | |
| | rPA in ng/ml*OD₆₀₀ | Stimulationsfaktor | rPA in ng/ml*OD₆₀₀ | Stimulationsfaktor | rPA in ng/ml*OD₆₀₀ | Stimulationsfaktor |
| **-** | 0,030 ± 0,001 | 29 | 0,044 ± 0,090 | 20 | 0,170 ± 0,005 | 23 |
| **DnaJ** | 0,197 ± 0,019 | 29 | 0,730 ± 0,150 | 27 | 3,978 ± 1,000 | 18 |
| **J-Domäne** | 0,339 ± 0,007 | 16 | 0,625 ± 0,213 | 17 | 4,398 ± 0,165 | 15 |
| **Hsp25** | 0,053 ± 0,002 | 27 | 0,140 ± 0,001 | 17 | 2,850 ± 0,214 | 17 |
| **ScFvOxazolon** | 0,041 ± 0,003 | 13 | 0,144 ± 0,047 | 8 | 0,713 ± 0,113 | 10 |

Die Kultivierung erfolgte in Gegenwart von 5 mM GSH.

**Tabelle 2:**

| **Expression von nativem rPA im Periplasma von E. coli bei Co-Sekretion von DnaJ und Zusatz verschiedener niedermolekularer faltungsverbessernder Zusätze** | | | | | |
|---|---|---|---|---|---|
| **Zusatz** | **Konzentration im Kulturmedium** | **Menge rPA in ng/ml*OD**_{**600**} **im Periplasma** | **Stimulationsfaktor** | **OD**_{**600**} **bei Zellernte** | **Konzentration GSH im Medium** |
| **ohne Zusätze** | - | 0,153 | 24 | 4,52 | 0 mM |
| **Arginin** | 0,2 M | 0,560 | 21 | 4,45 | 5 mM |
| | 0,4 M | 3,880 | 17 | 1,78 | 5 mM |
| **Formamid** | 0,6 M | 0,208 | 17 | 4,96 | 5 mM |
| | 1,0 M | 0,219 | 10 | 4,71 | 5 mM |
| **Methylformamid** | 0,3 M | 0,141 | 15 | 4,57 | 5 mM |
| | 0,6 M | 0,790 | 17 | 1,04 | 5 mM |
| **Acetamid** | 0,6 M | 0,150 | 24 | 5,34 | 5 mM |
| | 1,0 M | 1,321 | 16 | 1,57 | 5 mM |
| **Methylharnstoff** | 0,3 M | 0,168 | 24 | 4,67 | 5 mM |
| | 0,6 M | 0,830 | 22 | 4,59 | 5 mM |
| **Ethylharnstoff** | 0,3 M | 0,266 | 23 | 4,20 | 5 mM |
| | 0,6 M | 1,209 | 17 | 0,82 | 5 mM |

### Beispiel 10:

### Expression eines funktionellen Single-Chain-Fv-Fragmentes unter Zusatz von reduziertem Glutathion und L-Arginin zum Kulturmedium

Eine stationäre Übernachtkultur von E. coli BL21(DE3), die mit einem Plasmid, welches für ein Single-chain-Fᵥ-Fragment eines anti-TSH Antikörpers codiert und pUBS520 transformiert wurde, wurde im Verhältnis 1:50 in 100 ml LB-Medium mit Ampicillin (100 µg/ml) und Kanamycin (50 µg/ml, Fluka Chemica, Neu-Ulm) verdünnt und bei 24°C und 170 rpm geschüttelt. Nach 3 h Wachstum wurden je 5ml der Kultur zu je 10 ml LB-Medium mit o.g. Mengen an Ampicillin und Kanamycin und verschiedenen Konzentrationen von GSH (0-10 mM, Fluka) und L-Arginin HCl (0-0,4 M, ICN) gegeben und mit jeweils 1mM IPTG (Isopropyl-β-D-Thiogalactosid, AppliChem, Darmstadt) induziert. Die Zellen wurden weitere 21 h bei 24 °C und 170 rpm geschüttelt und nach Bestimmung der OD₆₀₀ eine 1 ml-Probe genommen. Diese 1 ml-Zellproben wurden nach einer modifizierten Vorschrift nach Jacobi et al. (J. Biol. Chem. 272 (1997) 21692-21699) in 2 ml-Eppendorf-Reaktionsgefäßen fraktioniert (s. Beispiel 8). Weiterhin wurde eine Probe des Medienüberstandes (1 ml) genommen. Zur Analyse der Proben auf funktionellen Antikörpern wurden sie einem ELISA-Test unterzogen.

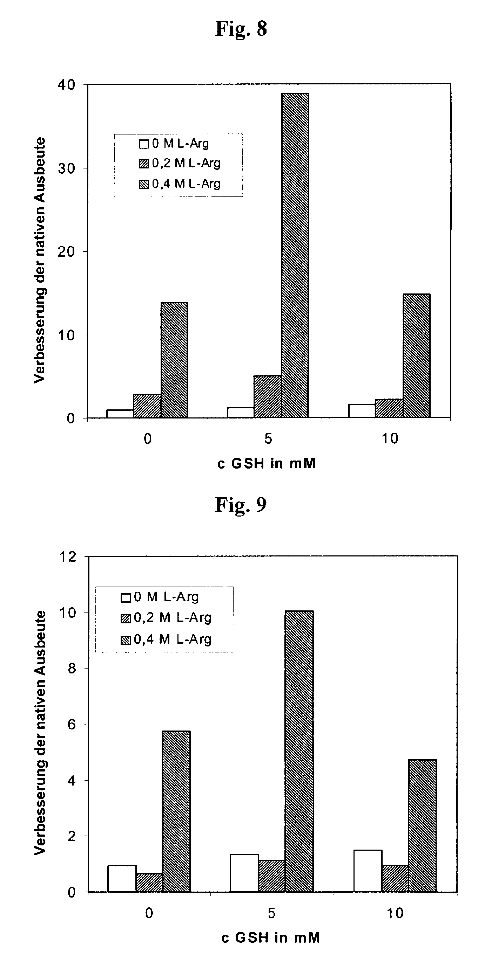

Der Zusatz von L-Arginin und GSH zum Kulturmedium im Falle der Expression von ScFv-TSH hatte ebenfalls einen positiven Einfluß auf die Ausbeute an nativem ScFv-TSH im Periplasma und im Medium-Überstand von E. coli. Bei Zugabe von 0,4 M L-Arginin und 5 mM GSH konnte die Menge an mittels ELISA detektiertem Antikörperfragment im Medien-Überstand um das 39-fache (Fig. 8) und in der periplasmatischen Fraktion um das 10-fache (Fig. 9) gegenüber einer Kultivierung ohne Medienzusätze gesteigert werden.

### Referenzliste

Allen et al., TIBS 20 (1995) 511-516
Arrigo & Landry (1994) In Morimoto (Hrsg.): The Biology of Heat Shock Proteins and Molecular Chaperones, Cold Spring Harbour Press, 335-373
Ausubel et al. (Hrsg.) Current Protocols in Molecular Biology, J. Wiley & Sons, 1997
Berges et al., Appl. Environ. Microbiol. 62 (1996) 55-60
Brinkmann et al., Gene 85 (1989) 109 - 114
Brinkmann et al., Proc. Natl. Acad. Sci USA 89 (1992) 3075-3079
Buchner & Rudolph, Bio/Technology 9 (1991)157-162
Bukau, B. & Horwich, A., Cell 92 (1998) 351-366
Ehrsperger et al., EMBO J. 16 (1997) 221-229
EP-A 0 510 658
EP-A 0 556 726
Fiedler und Conrad, Bio/Technology 13 (1995) 1090 - 1093
Gaestel et al., Eur. J. Biochem. 179 (1989) 209-213
Ghayreb et al., EMBO J. 3 (1984) 2437-2442
Goloubinoff et al., Nature 337 (1989) 44-47
Hockney, TIBTECH 12 (1994) 456- 463
Hudson, Curr. Opin Biotechnol. 9 (1998) 395-402
Jacobi et al. (J. Biol. Chem. 272 (1997) 21692-21699
Jakob et al., J. Biol. Chem. 268 (1993) 1517-1520
Kelley, TIBS 23 (1998) 222-227
Knappik et al., Bio/Technology 11(1993) 77-83
Kohnert et al., Protein Engineering 5 (1992) 93-100
Langer et al., Nature 356 (1992) 683 - 689
Lee & Olins, J. Biol. Chem. 267 (1992) 2849-2852
Lin & Traugh, Prot. Express. Purif. 4 (1993) 256-264).
Martin et al., Cardiovasc. Drug Rev. 11(1993) 299-311
Murphy & Beckwith: Export of Proteins to the Cell Envelope in Escherichia coli
Neidhardt et al. (Hrsg.): Escherichia coli and Salmonella, Second Edition, Vol. 1, ASM Press, Washington, 1996, S. 967-978
Paetzel et al., Nature 396(1998) 186- 190
Perez-Perez et al., Biochem. Biophys. Res. Commun. 210 (1995) 524-529
Qiu et al., Appl. Environm. Microbiol. 64 (1998) 4891 - 4896
Roman et al., Proc. Natl. Acad. Sci. USA 92 (1995) 8428-8432
Sato et al., Biochem. Biophys. Res. Commun. 202 (1994) 258-264
Schmidt et al., Prot. Engin. 11(1998) 601- 607
Schröder et al., EMBO J. 12 (1993) 4137-4144
Shibui et al., Appl. Microbiol. Biotechnol. 37 (1992) 352 - 357
Studier & Moffat, J. Mol. Biol. 189 (1986) 113-130
Thomas et al., Appl. Biochem. Biotechnol. 66 (1997) 197-238
US-Patent Nr. 5,223,256
US-Patent Nr. 5,593,865
Verheijen et al. Thromb. Haemostasis 48 (1982) 266-269
Wall et al., J. Biol. Chem. 270 (1995) 2139-2144
Yokoyama et al., Microbiol. Ferment. Technol. 62 (1998) 1205-1210

## Patentansprüche

1. Verfahren zur Herstellung eines wasserlöslichen, natürlich gefalteten eukaryontischen Polypeptids, enthaltend zwei oder mehrere über Disulfidbrücken verknüpfte Cysteine,
durch Kultivierung prokaryontischer Zellen,
a) wobei die genannten prokaryontischen Zellen einen Expressionsvektor enthalten, der für das genannte Polypeptid, das am N-Terminus eine prokaryontische Signalsequenz enthält, codiert,
b) unter Bedingungen, bei denen das Polypeptid in das Periplasma oder das Medium sekretiert wird,
c) Abspaltung der Signalsequenz und Isolierung des Polypeptids aus dem Periplasma oder dem Medium,
dadurch gekennzeichnet, daß die Kultivierung in Gegenwart von Arginin oder einer Verbindung der allgemeinen Formel I
R₂-CO-NRR₁ (I),
erfolgt, wobei R und R₁ Wasserstoff oder eine gesättigte oder ungesättigte verzweigte oder unverzweigte C₁-C₄-Alkylkette und
R₂ Wasserstoff, NHR₁ oder eine gesättigte oder ungesättigte verzweigte oder unverzweigte C₁-C₃-Alkylkette darstellen.

2. Verfahren nach Anspruch 1, dadurch gekennzeichnet, daß Arginin als Hydrochlorid oder als andere titrierte Form verwendet wird.

3. Verfahren nach den Ansprüchen 1 bis 2, dadurch gekennzeichnet, daß dem Nährmedium ein reduzierendes Thiolreagenz zugesetzt wird.

4. Verfahren nach Anspruch 3, dadurch gekennzeichnet, daß als reduzierendes Thiolreagenz Glutathion (GSH) verwendet wird.

5. Verfahren nach den Ansprüchen 1 bis 4, dadurch gekennzeichnet, daß die Signalsequenz aus gramnegativen Bakterien stammt.

6. Verfahren nach einem der Ansprüche 1 bis 5, dadurch gekennzeichnet, daß die prokaryontische Zelle einen weiteren Expressionsvektor, der für ein molekulares Chaperon codiert, enthält.

7. Verfahren nach Anspruch 6, dadurch gekennzeichnet, daß das molekulare Chaperon DNAJ aus E. coli oder HSP25 ist.

8. Verfahren nach einem der Ansprüche 6 oder 7, dadurch gekennzeichnet, daß die für das molekulare Chaperon codierende rekombinante DNA in operativer Verknüpfung mit einem DNA-Fragment steht, das ein Signalpeptid zum Durchdringen der inneren bakteriellen Membran codiert.

9. Verfahren nach einem der Ansprüche 6 bis 8, dadurch gekennzeichnet, daß sich die für das sekretierte molekulare Chaperon und/oder für das sekretierte Protein codierende DNA unter Kontrolle eines induzierbaren Expressionssignals befindet.

10. Verfahren nach einem der Ansprüche 1 bis 9, dadurch gekennzeichnet, daß das Polypeptid ein Antikörper, Antikörperfragment, Interferon, Proteinhormon oder eine Protease ist.


SEQUENZPROTOKOLL

<110>        F. Hoffmann-La Roche AG

<120>        Verfahren zur Herstellung von natuerlich
             gefalteten und sekretierten Proteinen

<130>        Case 20379

<160>        10

<210>        1
<211>        1881
<212>        DNA
<213>        E. coli

<220>
<221>        CDS
<222>        (392)...(1591)

<400>        1

TAGGCGTATC ACGAGGCCCT TTGGATAACC AGAAGCAATA AAAAATCAAA TCGGATTTCA        60

CTATATAATC TCACTTTATC TAAGATGAAT CCGATGGAAG CATCCTGTTT TCTCTCAATT       120

TTTTTATCTA AAACCCAGCG TTCGATGCTT CTTTGAGCGA ACGATCAAAA ATAAGTGCCT       180

TCCCATCAAA AAAATATTCT CAACATAAAA AACTTTGTGT AATACTTGTA ACGCTACATG       240

GAGATTAACT CAATCTAGCT AGAGAGGCTT TACACTTTAT GCTTCCGGCT CGTATAATGT       300

GTGGAATTGT GAGCGGATAA CAATTTCACA CAGGAAACAG CTATGACCAT GATTACGGAT       360

TCACTGGAAC TCTAGATAAC GAGGGCAAAA A ATG AAA AAG ACA GCT ATC GCG       412
                                   Met Lys Lys Thr Ala Ile Ala
                                    1               5

ATT GCA GTG GCA CTG GCT GGT TTC GCT ACC GTA GCG CAG GCC GGA ATT       460
Ile Ala Val Ala Leu Ala Gly Phe Ala Thr Val Ala Gln Ala Gly Ile
        10                  15                  20

CCA GCT AAG CAA GAT TAT TAC GAG ATT TTA GGC GTT TCC AAA ACA GCG       508
Pro Ala Lys Gln Asp Tyr Tyr Glu Ile Leu Gly Val Ser Lys Thr Ala
    25                  30                  35

GAA GAG CGT GAA ATC AGA AAG GCC TAC AAA CGC CTG GCC ATG AAA TAC       556
Glu Glu Arg Glu Ile Arg Lys Ala Tyr Lys Arg Leu Ala Met Lys Tyr
40                  45                  50                  55

CAC CCG GAC CGT AAC CAG GGT GAC AAA GAG GCC GAG GCG AAA TTT AAA       604
His Pro Asp Arg Asn Gln Gly Asp Lys Glu Ala Glu Ala Lys Phe Lys
                60                  65                  70

GAG ATC AAG GAA GCT TAT GAA GTT CTG ACC GAC TCG CAA AAA CGT GCG 652
Glu Ile Lys Glu Ala Tyr Glu Val Leu Thr Asp Ser Gln Lys Arg Ala
            75                  80                  85

GCA TAC GAT CAG TAT GGT CAT GCT GCG TTT GAG CAA GGT GGC ATG GGC 700
Ala Tyr Asp Gln Tyr Gly His Ala Ala Phe Glu Gln Gly Gly Met Gly
        90                  95                  100

GGC GGC GGT TTT GGC GGC GGC GCA GAC TTC AGC GAT ATT TTT GGT GAC 748
Gly Gly Gly Phe Gly Gly Gly Ala Asp Phe Ser Asp Ile Phe Gly Asp
    105                 110                 115

GTT TTC GGC GAT ATT TTT GGC GGC GGA CGT GGT CGT CAA CGT GCG GCG 796
Val Phe Gly Asp Ile Phe Gly Gly Gly Arg Gly Arg Gln Arg Ala Ala
120                 125                 130                 135

CGC GGT GCT GAT TTA CGC TAT AAC ATG GAG CTC ACC CTC GAA GAA GCT 844
Arg Gly Ala Asp Leu Arg Tyr Asn Met Glu Leu Thr Leu Glu Glu Ala
                140                 145                 150

GTA CGT GGC GTG ACC AAA GAG ATC CGC ATT CCG ACT CTG GAA GAG TGT 892
Val Arg Gly Val Thr Lys Glu Ile Arg Ile Pro Thr Leu Glu Glu Cys
            155                 160                 165

GAC GTT TGC CAC GGT AGC GGT GCA AAA CCA GGT ACA CAG CCG CAG ACT 940
Asp Val Cys His Gly Ser Gly Ala Lys Pro Gly Thr Gln Pro Gln Thr
        170                 175                 180

TGT CCG ACC TGT CAT GGT TCT GGT CAG GTG CAG ATG CGC CAG GGA TTC 988
Cys Pro Thr Cys His Gly Ser Gly Gln Val Gln Met Arg Gln Gly Phe
    185                 190                 195

TTC GCT GTA CAG CAG ACC TGT CCA CAC TGT CAG GGC CGC GGT ACG CTG 1036
Phe Ala Val Gln Gln Thr Cys Pro His Cys Gln Gly Arg Gly Thr Leu
200                 205                 210                 215

ATC AAA GAT CCG TGC AAC AAA TGT CAT GGT CAT GGT CGT GTT GAG CGC 1084
Ile Lys Asp Pro Cys Asn Lys Cys His Gly His Gly Arg Val Glu Arg
                220                 225                 230

AGC AAA ACG CTG TCC GTT AAA ATC CCG GCA GGG GTG GAC ACT GGA GAC 1132
Ser Lys Thr Leu Ser Val Lys Ile Pro Ala Gly Val Asp Thr Gly Asp
            235                 240                 245

CGC ATC CGT CTT GCG GGC GAA GGT GAA GCG GGC GAG CAT GGC GCA CCG 1180
Arg Ile Arg Leu Ala Gly Glu Gly Glu Ala Gly Glu His Gly Ala Pro
        250                 255                 260

GCA GGC GAT CTG TAC GTT CAG GTT CAG GTT AAA CAG CAC CCG ATT TTC 1228
Ala Gly Asp Leu Tyr Val Gln Val Gln Val Lys Gln His Pro Ile Phe
    265                 270                 275

GAG CGT GAA GGC AAC AAC CTG TAT TGC GAA GTC CCG ATC AAC TTC GCT 1276
Glu Arg Glu Gly Asn Asn Leu Tyr Cys Glu Val Pro Ile Asn Phe Ala
280                 285                 290                 295

ATG GCG GCG CTG GGT GGC GAA ATC GAA GTA CCG ACC CTT GAT GGT CGC 1324
Met Ala Ala Leu Gly Gly Glu Ile Glu Val Pro Thr Leu Asp Gly Arg
                300                 305                 310

GTC AAA CTG AAA GTG CCT GGC GAA ACC CAG ACC GGT AAG CTA TTC CGT 1372
Val Lys Leu Lys Val Pro Gly Glu Thr Gln Thr Gly Lys Leu Phe Arg
            315                 320                 325

ATG CGC GGT AAA GGC GTC AAG TCT GTC CGC GGT GGC GCA CAG GGT GAT 1420
Met Arg Gly Lys Gly Val Lys Ser Val Arg Gly Gly Ala Gln Gly Asp
        330                 335                 340

TTG CTG TGC CGC GTT GTC GTC GAA ACA CCG GTA GGC CTG AAC GAA AGG 1468
Leu Leu Cys Arg Val Val Val Glu Thr Pro Val Gly Leu Asn Glu Arg
    345                 350                 355

CAG AAA CAG CTG CTG CAA GAG CTG CAA GAA AGC TTC GGT GGC CCA ACC 1516
Gln Lys Gln Leu Leu Gln Glu Leu Gln Glu Ser Phe Gly Gly Pro Thr
360                 365                 370                 375

GGC GAG CAC AAC AGC CCG CGC TCA AAG AGC TTC TTT GAT GGT GTG AAG 1564
Gly Glu His Asn Ser Pro Arg Ser Lys Ser Phe Phe Asp Gly Val Lys
                380                 385                 390

AAG TTT TTT GAC GAC CTG ACC CGC TAA GGATCCGGCT GAGCAACGAC 1611
Lys Phe Phe Asp Asp Leu Thr Arg *
            395                 400

GTGAACGCAA TGCGTTCCGA CGTTCAGGCT GCTAAAGATG ACGCAGCTCG TGCTAACCAG 1671

CGTCTGGACA ACATGGCTAC TAAATACCGC AAGTAATAGT ACCTGTGAAG TGAAAAATGG 1731

CGCACATTGT GCGACATTTT TTTTGTCTGC CGTTTACCGC TACTGCGTCA CGCGTAACAT 1791

ATTCCCTTGC TCTGGTTCAC CATTCTGCGC TGACTCTACT GAAGGCGCAT TGCTGGCTGC 1851

GGGAGTTGCT CCACTGCTCA CCGAAACCGG 1881

<210> 2
<211> 400
<212> PRT
<213> E. coli

<400> 2

Met Lys Lys Thr Ala Ile Ala Ile Ala Val Ala Leu Ala Gly Phe Ala
1               5                   10                  15

Thr Val Ala Gln Ala Gly Ile Pro Ala Lys Gln Asp Tyr Tyr Glu Ile
            20                  25                  30

Leu Gly Val Ser Lys Thr Ala Glu Glu Arg Glu Ile Arg Lys Ala Tyr
        35                  40                  45

Lys Arg Leu Ala Met Lys Tyr His Pro Asp Arg Asn Gln Gly Asp Lys
    50                  55                  60

Glu Ala Glu Ala Lys Phe Lys Glu Ile Lys Glu Ala Tyr Glu Val Leu
65                  70                  75                  80

Thr Asp Ser Gln Lys Arg Ala Ala Tyr Asp Gln Tyr Gly His Ala Ala
                85                  90                  95

Phe Glu Gln Gly Gly Met Gly Gly Gly Gly Phe Gly Gly Gly Ala Asp
            100                 105                 110

Phe Ser Asp Ile Phe Gly Asp Val Phe Gly Asp Ile Phe Gly Gly Gly
        115                 120                 125

Arg Gly Arg Gln Arg Ala Ala Arg Gly Ala Asp Leu Arg Tyr Asn Met
    130                 135                 140

Glu Leu Thr Leu Glu Glu Ala Val Arg Gly Val Thr Lys Glu Ile Arg
145                 150                 155                 160

Ile Pro Thr Leu Glu Glu Cys Asp Val Cys His Gly Ser Gly Ala Lys
                165                 170                 175

Pro Gly Thr Gln Pro Gln Thr Cys Pro Thr Cys His Gly Ser Gly Gln
            180                 185                 190

Val Gln Met Arg Gln Gly Phe Phe Ala Val Gln Gln Thr Cys Pro His
        195                 200                 205

Cys Gln Gly Arg Gly Thr Leu Ile Lys Asp Pro Cys Asn Lys Cys His
    210                 215                 220

Gly His Gly Arg Val Glu Arg Ser Lys Thr Leu Ser Val Lys Ile Pro
225                 230                 235                 240

Ala Gly Val Asp Thr Gly Asp Arg Ile Arg Leu Ala Gly Glu Gly Glu
                245                 250                 255

Ala Gly Glu His Gly Ala Pro Ala Gly Asp Leu Tyr Val Gln Val Gln
            260                 265                 270

Val Lys Gln His Pro Ile Phe Glu Arg Glu Gly Asn Asn Leu Tyr Cys
        275                 280                 285

Glu Val Pro Ile Asn Phe Ala Met Ala Ala Leu Gly Gly Glu Ile Glu
    290                 295                 300

Val Pro Thr Leu Asp Gly Arg Val Lys Leu Lys Val Pro Gly Glu Thr
305                 310                 315                 320

Gln Thr Gly Lys Leu Phe Arg Met Arg Gly Lys Gly Val Lys Ser Val
                325                 330                 335

Arg Gly Gly Ala Gln Gly Asp Leu Leu Cys Arg Val Val Val Glu Thr
            340                 345                 350

Pro Val Gly Leu Asn Glu Arg Gln Lys Gln Leu Leu Gln Glu Leu Gln
        355                 360                 365

Glu Ser Phe Gly Gly Pro Thr Gly Glu His Asn Ser Pro Arg Ser Lys
    370                 375                 380

Ser Phe Phe Asp Gly Val Lys Lys Phe Phe Asp Asp Leu Thr Arg  *
385                 390                 395                 400

<210>       3
<211>       1881
<212>       DNA
<213>       E. coli

<220>
<221>       CDS
<222>       (392)...(790)

<400>       3

TAGGCGTATC ACGAGGCCCT TTGGATAACC AGAAGCAATA AAAAATCAAA TCGGATTTCA      60

CTATATAATC TCACTTTATC TAAGATGAAT CCGATGGAAG CATCCTGTTT TCTCTCAATT     120

TTTTTATCTA AAACCCAGCG TTCGATGCTT CTTTGAGCGA ACGATCAAAA ATAAGTGCCT     180

TCCCATCAAA AAAATATTCT CAACATAAAA AACTTTGTGT AATACTTGTA ACGCTACATG     240

GAGATTAACT CAATCTAGCT AGAGAGGCTT TACACTTTAT GCTTCCGGCT CGTATAATGT     300

GTGGAATTGT GAGCGGATAA CAATTTCACA CAGGAAACAG CTATGACCAT GATTACGGAT     360

TCACTGGAAC TCTAGATAAC GAGGGCAAAA A ATG AAA AAG ACA GCT ATC GCG     412
                                   Met Lys Lys Thr Ala Ile Ala
                                     1               5

ATT GCA GTG GCA CTG GCT GGT TTC GCT ACC GTA GCG CAG GCC GGA ATT     460
Ile Ala Val Ala Leu Ala Gly Phe Ala Thr Val Ala Gln Ala Gly Ile
        10                  15                  20

CCA GCT AAG CAA GAT TAT TAC GAG ATT TTA GGC GTT TCC AAA ACA GCG     508
Pro Ala Lys Gln Asp Tyr Tyr Glu Ile Leu Gly Val Ser Lys Thr Ala
    25                  30                  35

GAA GAG CGT GAA ATC AGA AAG GCC TAC AAA CGC CTG GCC ATG AAA TAC     556
Glu Glu Arg Glu Ile Arg Lys Ala Tyr Lys Arg Leu Ala Met Lys Tyr
 40                  45                  50                  55

CAC CCG GAC CGT AAC CAG GGT GAC AAA GAG GCC GAG GCG AAA TTT AAA     604
His Pro Asp Arg Asn Gln Gly Asp Lys Glu Ala Glu Ala Lys Phe Lys
                60                  65                  70

GAG ATC AAG GAA GCT TAT GAA GTT CTG ACC GAC TCG CAA AAA CGT GCG 652
Glu Ile Lys Glu Ala Tyr Glu Val Leu Thr Asp Ser Gln Lys Arg Ala
            75                  80                  85

GCA TAC GAT CAG TAT GGT CAT GCT GCG TTT GAG CAA GGT GGC ATG GGC 700
Ala Tyr Asp Gln Tyr Gly His Ala Ala Phe Glu Gln Gly Gly Met Gly
        90                  95                  100

GGC GGC GGT TTT GGC GGC GGC GCA GAC TTC AGC GAT ATT TTT GGT GAC 748
Gly Gly Gly Phe Gly Gly Gly Ala Asp Phe Ser Asp Ile Phe Gly Asp
    105                 110                 115

GTT TTC GGC GAT ATT TTT GGC GGC GGA CGT GGT CGT TAA TAG 790
Val Phe Gly Asp Ile Phe Gly Gly Gly Arg Gly Arg * *
120                 125                 130

GCGGCGCGCG GTGCTGATTT ACGCTATAAC ATGGAGCTCA CCCTCGAAGA AGCTGTACGT 850

GGCGTGACCA AAGAGATCCG CATTCCGACT CTGGAAGAGT GTGACGTTTG CCACGGTAGC 910

GGTGCAAAAC CAGGTACACA GCCGCAGACT TGTCCGACCT GTCATGGTTC TGGTCAGGTG 970

CAGATGCGCC AGGGATTCTT CGCTGTACAG CAGACCTGTC CACACTGTCA GGGCCGCGGT 1030

ACGCTGATCA AAGATCCGTG CAACAAATGT CATGGTCATG GTCGTGTTGA GCGCAGCAAA 1090

ACGCTGTCCG TTAAAATCCC GGCAGGGGTG GACACTGGAG ACCGCATCCG TCTTGCGGGC 1150

GAAGGTGAAG CGGGCGAGCA TGGCGCACCG GCAGGCGATC TGTACGTTCA GGTTCAGGTT 1210

AAACAGCACC CGATTTTCGA GCGTGAAGGC AACAACCTGT ATTGCGAAGT CCCGATCAAC 1270

TTCGCTATGG CGGCGCTGGG TGGCGAAATC GAAGTACCGA CCCTTGATGG TCGCGTCAAA 1330

CTGAAAGTGC CTGGCGAAAC CCAGACCGGT AAGCTATTCC GTATGCGCGG TAAAGGCGTC 1390

AAGTCTGTCC GCGGTGGCGC ACAGGGTGAT TTGCTGTGCC GCGTTGTCGT CGAAACACCG 1450

GTAGGCCTGA ACGAAAGGCA GAAACAGCTG CTGCAAGAGC TGCAAGAAAG CTTCGGTGGC 1510

CCAACCGGCG AGCACAACAG CCCGCGCTCA AAGAGCTTCT TTGATGGTGT GAAGAAGTTT 1570

TTTGACGACC TGACCCGCTA AGGATCCGGC TGAGCAACGA CGTGAACGCA ATGCGTTCCG 1630

ACGTTCAGGC TGCTAAAGAT GACGCAGCTC GTGCTAACCA GCGTCTGGAC AACATGGCTA 1690

CTAAATACCG CAAGTAATAG TACCTGTGAA GTGAAAAATG GCGCACATTG TGCGACATTT 1750

TTTTTGTCTG CCGTTTACCG CTACTGCGTC ACGCGTAACA TATTCCCTTG CTCTGGTTCA 1810

CCATTCTGCG CTGACTCTAC TGAAGGCGCA TTGCTGGCTG CGGGAGTTGC TCCACTGCTC 1870

ACCGAAACCG G 1881

<210>	4
<211>	133
<212>	PRT
<213>	E. coli

<400>	4

Met Lys Lys Thr Ala Ile Ala Ile Ala Val Ala Leu Ala Gly Phe Ala
1               5                   10                  15

Thr Val Ala Gln Ala Gly Ile Pro Ala Lys Gln Asp Tyr Tyr Glu Ile
            20                  25                  30

Leu Gly Val Ser Lys Thr Ala Glu Glu Arg Glu Ile Arg Lys Ala Tyr
        35                  40                  45

Lys Arg Leu Ala Met Lys Tyr His Pro Asp Arg Asn Gln Gly Asp Lys
    50                  55                  60

Glu Ala Glu Ala Lys Phe Lys Glu Ile Lys Glu Ala Tyr Glu Val Leu
65                  70                  75                  80

Thr Asp Ser Gln Lys Arg Ala Ala Tyr Asp Gln Tyr Gly His Ala Ala
                85                  90                  95

Phe Glu Gln Gly Gly Met Gly Gly Gly Gly Phe Gly Gly Gly Ala Asp
            100                 105                 110

Phe Ser Asp Ile Phe Gly Asp Val Phe Gly Asp Ile Phe Gly Gly Gly
        115                 120                 125

Arg Gly Arg  *   *
    130

<210>	5
<211>	1379
<212>	DNA
<213>	E. coli

<220>
<221>	CDS
<222>	(392)...(1090)

<400>	5

TAGGCGTATC ACGAGGCCCT TTGGATAACC AGAAGCAATA AAAAATCAAA TCGGATTTCA	60

CTATATAATC TCACTTTATC TAAGATGAAT CCGATGGAAG CATCCTGTTT TCTCTCAATT	120

TTTTTATCTA AAACCCAGCG TTCGATGCTT CTTTGAGCGA ACGATCAAAA ATAAGTGCCT	180

TCCCATCAAA AAAATATTCT CAACATAAAA AACTTTGTGT AATACTTGTA ACGCTACATG	240

GAGATTAACT CAATCTAGCT AGAGAGGCTT TACACTTTAT GCTTCCGGCT CGTATAATGT	300

GTGGAATTGT GAGCGGATAA CAATTTCACA CAGGAAACAG CTATGACCAT GATTACGGAT 360

TCACTGGAAC TCTAGATAAC GAGGGCAAAA A ATG AAA AAG ACA GCT ATC GCG 412
Met Lys Lys Thr Ala Ile Ala
1               5

ATT GCA GTG GCA CTG GCT GGT TTC GCT ACC GTA GCG CAG GCC GGA ATT 460
Ile Ala Val Ala Leu Ala Gly Phe Ala Thr Val Ala Gln Ala Gly Ile
        10                  15                  20

CTC ACC GAG CGC CGC GTG CCC TTC TCG CTG CTG CGG AGC CCG AGC TGG 508
Leu Thr Glu Arg Arg Val Pro Phe Ser Leu Leu Arg Ser Pro Ser Trp
    25                  30                  35

GAA CCA TTC CGG GAC TGG TAC CCT GCA CAC AGC CGC CTC TTC GAT CAA 556
Glu Pro Phe Arg Asp Trp Tyr Pro Ala His Ser Arg Leu Phe Asp Gln
40                  45                  50                  55

GCT TTC GGG GTG CCC CGG TTG CCC GAT GAG TGG TCG CAG TGG TTC AGC 604
Ala Phe Gly Val Pro Arg Leu Pro Asp Glu Trp Ser Gln Trp Phe Ser
                60                  65                  70

GCC GCT GGG TGG CCC GGA TAC GTG CGC CCG CTG CCC GCC GCG ACC GCC 652
Ala Ala Gly Trp Pro Gly Tyr Val Arg Pro Leu Pro Ala Ala Thr Ala
            75                  80                  85

GAG GGC CCC GCG GCG GTG ACC CTG GCC GCA CCA GCC TTC AGC CGA GCG 700
Glu Gly Pro Ala Ala Val Thr Leu Ala Ala Pro Ala Phe Ser Arg Ala
        90                  95                  100

CTC AAC CGA CAG CTC AGC AGC GGG GTC TCG GAG ATC CGA CAG ACG GCT 748
Leu Asn Arg Gln Leu Ser Ser Gly Val Ser Glu Ile Arg Gln Thr Ala
    105                 110                 115

GAT CGC TGG CGC GTG TCC CTG GAC GTC AAC CAC TTC GCT CCG GAG GAG 796
Asp Arg Trp Arg Val Ser Leu Asp Val Asn His Phe Ala Pro Glu Glu
120                 125                 130                 135

CTC ACA GTG AAG ACC AAG GAA GGC GTG GTG GAG ATC ACT GGC AAG CAC 844
Leu Thr Val Lys Thr Lys Glu Gly Val Val Glu Ile Thr Gly Lys His
                140                 145                 150

GAA GAA AGG CAG GAC GAA CAT GGC TAC ATC TCT CGG TGC TTC ACC CGG 892
Glu Glu Arg Gln Asp Glu His Gly Tyr Ile Ser Arg Cys Phe Thr Arg
            155                 160                 165

AAA TAC ACG CTC CCT CCA GGT GTG GAC CCC ACC CTA GTG TCC TCT TCC 940
Lys Tyr Thr Leu Pro Pro Gly Val Asp Pro Thr Leu Val Ser Ser Ser
        170                 175                 180

CTA TCC CCT GAG GGC ACA CTT ACC GTG GAG GCT CCG TTG CCC AAA GCA 988
Leu Ser Pro Glu Gly Thr Leu Thr Val Glu Ala Pro Leu Pro Lys Ala
    185                 190                 195

GTC ACG CAG TCA GCG GAG ATC ACC ATT CCG GTT ACT TTC GAG GCC CGC     1036
Val Thr Gln Ser Ala Glu Ile Thr Ile Pro Val Thr Phe Glu Ala Arg
200                 205                 210                 215

GCC CAA ATT GGG GGC CCA GAA GCT GGG AAG TCT GAA CAG TCT GGA GCC     1084
Ala Gln Ile Gly Gly Pro Glu Ala Gly Lys Ser Glu Gln Ser Gly Ala
                220                 225                 230

AAG TAG GATCCGGCTG AGCAACGACG TGAACGCAAT GCGTTCCGAC GTTCAGGCTG     1140
Lys  *

CTAAAGATGA CGCAGCTCGT GCTAACCAGC GTCTGGACAA CATGGCTACT AAATACCGCA     1200

AGTAATAGTA CCTGTGAAGT GAAAAATGGC GCACATTGTG CGACATTTTT TTTGTCTGCC     1260

GTTTACCGCT ACTGCGTCAC GCGTAACATA TTCCCTTGCT CTGGTTCACC ATTCTGCGCT     1320

GACTCTACTG AAGGCGCATT GCTGGCTGCG GGAGTTGCTC CACTGCTCAC CGAAACCGG     1379

<210>     6
<211>     233
<212>     PRT
<213>     E. coli

<400>     6

Met Lys Lys Thr Ala Ile Ala Ile Ala Val Ala Leu Ala Gly Phe Ala
  1               5                  10                  15

Thr Val Ala Gln Ala Gly Ile Leu Thr Glu Arg Arg Val Pro Phe Ser
             20                  25                  30

Leu Leu Arg Ser Pro Ser Trp Glu Pro Phe Arg Asp Trp Tyr Pro Ala
         35                  40                  45

His Ser Arg Leu Phe Asp Gln Ala Phe Gly Val Pro Arg Leu Pro Asp
     50                  55                  60

Glu Trp Ser Gln Trp Phe Ser Ala Ala Gly Trp Pro Gly Tyr Val Arg
 65                  70                  75                  80

Pro Leu Pro Ala Ala Thr Ala Glu Gly Pro Ala Ala Val Thr Leu Ala
                 85                  90                  95

Ala Pro Ala Phe Ser Arg Ala Leu Asn Arg Gln Leu Ser Ser Gly Val
            100                 105                 110

Ser Glu Ile Arg Gln Thr Ala Asp Arg Trp Arg Val Ser Leu Asp Val
        115                 120                 125

Asn His Phe Ala Pro Glu Glu Leu Thr Val Lys Thr Lys Glu Gly Val
    130                 135                 140

Val Glu Ile Thr Gly Lys His Glu Glu Arg Gln Asp Glu His Gly Tyr
145                 150                 155                 160

Ile Ser Arg Cys Phe Thr Arg Lys Tyr Thr Leu Pro Pro Gly Val Asp
                165                 170                 175

Pro Thr Leu Val Ser Ser Ser Leu Ser Pro Glu Gly Thr Leu Thr Val
            180                 185                 190

Glu Ala Pro Leu Pro Lys Ala Val Thr Gln Ser Ala Glu Ile Thr Ile
        195                 200                 205

Pro Val Thr Phe Glu Ala Arg Ala Gln Ile Gly Gly Pro Glu Ala Gly
    210                 215                 220

Lys Ser Glu Gln Ser Gly Ala Lys  *
225                 230

<210>       7
<211>       1256
<212>       DNA
<213>       E. coli

<220>
<221>       CDS
<222>       (199)...(969)

<400>       7

GATCTGGCTT TACACTTTAT GCTTCCGGCT CGTATGTTGT GTGGAATTGT GAGCGGATAA       60

CAATTTCACA CAGGAAACAG CTATGACCAT GATTACGCCA AGCTTGCATG CAAATTCTAT      120

TTCAAGGAGA CAGTCATAAT GAAATACCTA TTGCCTACGG CAGCCGCTGG ATTGTTATTA      180

CTCGCGGCCC AGCCGGCC ATG GCC GAG GTC AAG CTG CAG GAG TCT GGG GGA        231
                    Met Ala Glu Val Lys Leu Gln Glu Ser Gly Gly
                     1               5                  10

GGC TTA GTG CAG CCT GGA GGG TCC CGG AAA CTC TCC TGT GCA GCC TCT        279
Gly Leu Val Gln Pro Gly Gly Ser Arg Lys Leu Ser Cys Ala Ala Ser
            15                  20                  25

GGA TTC ACT TTC AGT AGC TTT GGA ATG CAC TGG GTT CGT CAG GCT CCA        327
Gly Phe Thr Phe Ser Ser Phe Gly Met His Trp Val Arg Gln Ala Pro
        30                  35                  40

GAG AAG GGG CTG GAG TGG GTC GCA TAT ATT AGT AGT GGC AGT AGT ACC        375
Glu Lys Gly Leu Glu Trp Val Ala Tyr Ile Ser Ser Gly Ser Ser Thr
    45                  50                  55

ATC TAC TAT GCA GAC ACA GTG AAG GGC CGA TTC ACC ATC TCC AGA GAC        423
Ile Tyr Tyr Ala Asp Thr Val Lys Gly Arg Phe Thr Ile Ser Arg Asp
60                  65                  70                  75

AAT CCC AAG AAC ACC CTG TTC CTG CAA ATG ACC AGT CTA AGG TCT GAG     471
Asn Pro Lys Asn Thr Leu Phe Leu Gln Met Thr Ser Leu Arg Ser Glu
                80                  85                  90

GAC ACG GCC ATG TAT TAC TGC GCA AGA GAT TAC GGG GCT TAT TGG GGC     519
Asp Thr Ala Met Tyr Tyr Cys Ala Arg Asp Tyr Gly Ala Tyr Trp Gly
            95                  100                 105

CAA GGG ACC ACG GTC ACC GTC TCC TCA GGT GGA GGC GGT TCA GGC GGA     567
Gln Gly Thr Thr Val Thr Val Ser Ser Gly Gly Gly Gly Ser Gly Gly
        110                 115                 120

GGT GGC TCT GGC GGT GGC GGA TCG GAC ATT GAG CTC ACC CAG TCT CCA     615
Gly Gly Ser Gly Gly Gly Gly Ser Asp Ile Glu Leu Thr Gln Ser Pro
    125                 130                 135

GCA ATC ATG TCT GCA TCT CCA GGG GAG AAG GTC ACC ATG ACC TGC AGT     663
Ala Ile Met Ser Ala Ser Pro Gly Glu Lys Val Thr Met Thr Cys Ser
140                 145                 150                 155

GCC AGT TCA AGT GTA AGG TAC ATG AAC TGG TTC CAA CAG AAG TCA GGC     711
Ala Ser Ser Ser Val Arg Tyr Met Asn Trp Phe Gln Gln Lys Ser Gly
                160                 165                 170

ACC TCC CCC AAA AGA TGG ATT TAT GAC ACA TCC AAA CTG TCT TCT GGA     759
Thr Ser Pro Lys Arg Trp Ile Tyr Asp Thr Ser Lys Leu Ser Ser Gly
            175                 180                 185

GTC CCT GCT CGC TTC AGT GGC AGT GGG TCT GGG ACC TCT TAC TCT CTC     807
Val Pro Ala Arg Phe Ser Gly Ser Gly Ser Gly Thr Ser Tyr Ser Leu
        190                 195                 200

ACA ATC AGC AGC ATG GAG GCT GAA GAT GCT GCC ACT TAT TAC TGC CAG     855
Thr Ile Ser Ser Met Glu Ala Glu Asp Ala Ala Thr Tyr Tyr Cys Gln
    205                 210                 215

CAG TGG AGT AGT AAT CCA CTC ACT TTC GGT GCT GGG ACC AAG CTG GAG     903
Gln Trp Ser Ser Asn Pro Leu Thr Phe Gly Ala Gly Thr Lys Leu Glu
220                 225                 230                 235

CTG AAA CGG GCG GCC GCA GAA CAA AAA CTC ATC TCA GAA GAG GAT CTG     951
Leu Lys Arg Ala Ala Ala Glu Gln Lys Leu Ile Ser Glu Glu Asp Leu
                240                 245                 250

AAT GGG GCC GCA TAG TAA CTGAGCAACG ACGTGAACGC AATGCGTTCC     999
Asn Gly Ala Ala  *   *
            255

GACGTTCAGG CTGCTAAAGA TGACGCAGCT CGTGCTAACC AGCGTCTGGA CAACATGGCT     1059

ACTAAATACC GCAAGTAATA GTACCTGTGA AGTGAAAAAT GGCGCACATT GTGCGACATT     1119

TTTTTTGTCT GCCGTTTACC GCTACTGCGT CACGCGTAAC ATATTCCCTT GCTCTGGTTC     1179

ACCATTCTGC GCTGACTCTA CTGAAGGCGC ATTGCTGGCT GCGGGAGTTG CTCCACTGCT     1239

CACCGAAACC GGAGATC                                                    1256

<210>    8
<211>    257
<212>    PRT
<213>    E. coli

<400>    8

Met Ala Glu Val Lys Leu Gln Glu Ser Gly Gly Gly Leu Val Gln Pro
1               5                   10                  15

Gly Gly Ser Arg Lys Leu Ser Cys Ala Ala Ser Gly Phe Thr Phe Ser
            20                  25                  30

Ser Phe Gly Met His Trp Val Arg Gln Ala Pro Glu Lys Gly Leu Glu
        35                  40                  45

Trp Val Ala Tyr Ile Ser Ser Gly Ser Ser Thr Ile Tyr Tyr Ala Asp
    50                  55                  60

Thr Val Lys Gly Arg Phe Thr Ile Ser Arg Asp Asn Pro Lys Asn Thr
65                  70                  75                  80

Leu Phe Leu Gln Met Thr Ser Leu Arg Ser Glu Asp Thr Ala Met Tyr
                85                  90                  95

Tyr Cys Ala Arg Asp Tyr Gly Ala Tyr Trp Gly Gln Gly Thr Thr Val
            100                 105                 110

Thr Val Ser Ser Gly Gly Gly Gly Ser Gly Gly Gly Gly Ser Gly Gly
        115                 120                 125

Gly Gly Ser Asp Ile Glu Leu Thr Gln Ser Pro Ala Ile Met Ser Ala
    130                 135                 140

Ser Pro Gly Glu Lys Val Thr Met Thr Cys Ser Ala Ser Ser Ser Val
145                 150                 155                 160

Arg Tyr Met Asn Trp Phe Gln Gln Lys Ser Gly Thr Ser Pro Lys Arg
                165                 170                 175

Trp Ile Tyr Asp Thr Ser Lys Leu Ser Ser Gly Val Pro Ala Arg Phe
            180                 185                 190

Ser Gly Ser Gly Ser Gly Thr Ser Tyr Ser Leu Thr Ile Ser Ser Met
        195                 200                 205

Glu Ala Glu Asp Ala Ala Thr Tyr Tyr Cys Gln Gln Trp Ser Ser Asn
    210                 215                 220

Pro Leu Thr Phe Gly Ala Gly Thr Lys Leu Glu Leu Lys Arg Ala Ala
225                 230                 235                 240

Ala Glu Gln Lys Leu Ile Ser Glu Glu Asp Leu Asn Gly Ala Ala *
                245                 250                 255

*

<210> 9
<211> 1137
<212> DNA
<213> E. coli

<220>
<221> CDS
<222> (1)...(1137)

<400> 9

ATG AAA TAC CTG CTG CCG ACC GCT GCT GCT GGT CTG CTG CTC CTC GCT 48
Met Lys Tyr Leu Leu Pro Thr Ala Ala Ala Gly Leu Leu Leu Leu Ala
1               5                   10                  15

GCC CAG CCG GCG ATG GCC ATG GCT TAC CAA GGA AAC AGT GAC TGC TAC 96
Ala Gln Pro Ala Met Ala Met Ala Tyr Gln Gly Asn Ser Asp Cys Tyr
            20                  25                  30

TTT GGG AAT GGG TCA GCC TAC CGT GGC ACG CAC AGC CTC ACC GAG TCG 144
Phe Gly Asn Gly Ser Ala Tyr Arg Gly Thr His Ser Leu Thr Glu Ser
        35                  40                  45

GGT GCC TCC TGC CTC CCG TGG AAT TCC ATG ATC CTG ATA GGC AAG GTT 192
Gly Ala Ser Cys Leu Pro Trp Asn Ser Met Ile Leu Ile Gly Lys Val
    50                  55                  60

TAC ACA GCA CAG AAC CCC AGT GCC CAG GCA CTG GGC CTG GGC AAA CAT 240
Tyr Thr Ala Gln Asn Pro Ser Ala Gln Ala Leu Gly Leu Gly Lys His
65                  70                  75                  80

AAT TAC TGC CGG AAT CCT GAT GGG GAT GCC AAG CCC TGG TGC CAC GTG 288
Asn Tyr Cys Arg Asn Pro Asp Gly Asp Ala Lys Pro Trp Cys His Val
                85                  90                  95

CTG ACG AAC CGC AGG CTG ACG TGG GAG TAC TGT GAT GTG CCC TCC TGC 336
Leu Thr Asn Arg Arg Leu Thr Trp Glu Tyr Cys Asp Val Pro Ser Cys
            100                 105                 110

TCC ACC TGC GGC CTG AGA CAG TAC AGC CAG CCT CAG TTT CGC ATC AAA 384
Ser Thr Cys Gly Leu Arg Gln Tyr Ser Gln Pro Gln Phe Arg Ile Lys
        115                 120                 125

GGA GGG CTC TTC GCC GAC ATC GCC TCC CAC CCC TGG CAG GCT GCC ATC 432
Gly Gly Leu Phe Ala Asp Ile Ala Ser His Pro Trp Gln Ala Ala Ile
    130                 135                 140

TTT GCC AAG CAC AGG AGG TCG CCC GGA GAG CGG TTC CTG TGC GGG GGC     480
Phe Ala Lys His Arg Arg Ser Pro Gly Glu Arg Phe Leu Cys Gly Gly
145                 150                 155                 160

ATA CTC ATC AGC TCC TGC TGG ATT CTC TCT GCC GCC CAC TGC TTC CAG     528
Ile Leu Ile Ser Ser Cys Trp Ile Leu Ser Ala Ala His Cys Phe Gln
                165                 170                 175

GAG AGG TTT CCG CCC CAC CAC CTG ACG GTG ATC TTG GGC AGA ACA TAC     576
Glu Arg Phe Pro Pro His His Leu Thr Val Ile Leu Gly Arg Thr Tyr
            180                 185                 190

CGG GTG GTC CCT GGC GAG GAG GAG CAG AAA TTT GAA GTC GAA AAA TAC     624
Arg Val Val Pro Gly Glu Glu Glu Gln Lys Phe Glu Val Glu Lys Tyr
        195                 200                 205

ATT GTC CAT AAG GAA TTC GAT GAT GAC ACT TAC GAC AAT GAC ATT GCG     672
Ile Val His Lys Glu Phe Asp Asp Asp Thr Tyr Asp Asn Asp Ile Ala
    210                 215                 220

CTG CTG CAG CTG AAA TCG GAT TCG TCC CGC TGT GCC CAG GAG AGC AGC     720
Leu Leu Gln Leu Lys Ser Asp Ser Ser Arg Cys Ala Gln Glu Ser Ser
225                 230                 235                 240

GTG GTC CGC ACT GTG TGC CTT CCC CCG GCG GAC CTG CAG CTG CCG GAC     768
Val Val Arg Thr Val Cys Leu Pro Pro Ala Asp Leu Gln Leu Pro Asp
                245                 250                 255

TGG ACG GAG TGT GAG CTC TCC GGC TAC GGC AAG CAT GAG GCC TTG TCT     816
Trp Thr Glu Cys Glu Leu Ser Gly Tyr Gly Lys His Glu Ala Leu Ser
            260                 265                 270

CCT TTC TAT TCG GAG CGG CTG AAG GAG GCT CAT GTC AGA CTG TAC CCA     864
Pro Phe Tyr Ser Glu Arg Leu Lys Glu Ala His Val Arg Leu Tyr Pro
        275                 280                 285

TCC AGC CGC TGC ACA TCA CAA CAT TTA CTT AAC AGA ACA GTC ACC GAC     912
Ser Ser Arg Cys Thr Ser Gln His Leu Leu Asn Arg Thr Val Thr Asp
    290                 295                 300

AAC ATG CTG TGT GCT GGA GAC ACT CGG AGC GGC GGG CCC CAG GCA AAC     960
Asn Met Leu Cys Ala Gly Asp Thr Arg Ser Gly Gly Pro Gln Ala Asn
305                 310                 315                 320

TTG CAC GAC GCC TGC CAG GGC GAT TCG GGA GGC CCC CTG GTG TGT CTG     1008
Leu His Asp Ala Cys Gln Gly Asp Ser Gly Gly Pro Leu Val Cys Leu
                325                 330                 335

AAC GAT GGC CGC ATG ACT TTG GTG GGC ATC ATC AGC TGG GGC CTG GGC     1056
Asn Asp Gly Arg Met Thr Leu Val Gly Ile Ile Ser Trp Gly Leu Gly
            340                 345                 350

TGT GGA CAG AAG GAT GTC CCG GGT GTG TAC ACC AAG GTT ACC AAC TAC     1104
Cys Gly Gln Lys Asp Val Pro Gly Val Tyr Thr Lys Val Thr Asn Tyr
        355                 360                 365

CTA GAC TGG ATT CGT GAC AAC ATG CGA CCG TGA                                    1137
Leu Asp Trp Ile Arg Asp Asn Met Arg Pro  *
    370                 375

<210>       10
<211>       379
<212>       PRT
<213>       E. coli

<400>       10

Met Lys Tyr Leu Leu Pro Thr Ala Ala Ala Gly Leu Leu Leu Leu Ala
  1               5                  10                  15

Ala Gln Pro Ala Met Ala Met Ala Tyr Gln Gly Asn Ser Asp Cys Tyr
             20                  25                  30

Phe Gly Asn Gly Ser Ala Tyr Arg Gly Thr His Ser Leu Thr Glu Ser
         35                  40                  45

Gly Ala Ser Cys Leu Pro Trp Asn Ser Met Ile Leu Ile Gly Lys Val
     50                  55                  60

Tyr Thr Ala Gln Asn Pro Ser Ala Gln Ala Leu Gly Leu Gly Lys His
 65                  70                  75                  80

Asn Tyr Cys Arg Asn Pro Asp Gly Asp Ala Lys Pro Trp Cys His Val
                 85                  90                  95

Leu Thr Asn Arg Arg Leu Thr Trp Glu Tyr Cys Asp Val Pro Ser Cys
            100                 105                 110

Ser Thr Cys Gly Leu Arg Gln Tyr Ser Gln Pro Gln Phe Arg Ile Lys
        115                 120                 125

Gly Gly Leu Phe Ala Asp Ile Ala Ser His Pro Trp Gln Ala Ala Ile
    130                 135                 140

Phe Ala Lys His Arg Arg Ser Pro Gly Glu Arg Phe Leu Cys Gly Gly
145                 150                 155                 160

Ile Leu Ile Ser Ser Cys Trp Ile Leu Ser Ala Ala His Cys Phe Gln
                165                 170                 175

Glu Arg Phe Pro Pro His His Leu Thr Val Ile Leu Gly Arg Thr Tyr
            180                 185                 190

Arg Val Val Pro Gly Glu Glu Glu Gln Lys Phe Glu Val Glu Lys Tyr
        195                 200                 205

Ile Val His Lys Glu Phe Asp Asp Asp Thr Tyr Asp Asn Asp Ile Ala
    210                 215                 220

Leu Leu Gln Leu Lys Ser Asp Ser Ser Arg Cys Ala Gln Glu Ser Ser
225                 230                 235                 240

Val Val Arg Thr Val Cys Leu Pro Pro Ala Asp Leu Gln Leu Pro Asp
                245                 250                 255

Trp Thr Glu Cys Glu Leu Ser Gly Tyr Gly Lys His Glu Ala Leu Ser
            260                 265                 270

Pro Phe Tyr Ser Glu Arg Leu Lys Glu Ala His Val Arg Leu Tyr Pro
        275                 280                 285

Ser Ser Arg Cys Thr Ser Gln His Leu Leu Asn Arg Thr Val Thr Asp
    290                 295                 300

Asn Met Leu Cys Ala Gly Asp Thr Arg Ser Gly Gly Pro Gln Ala Asn
305                 310                 315                 320

Leu His Asp Ala Cys Gln Gly Asp Ser Gly Gly Pro Leu Val Cys Leu
                325                 330                 335

Asn Asp Gly Arg Met Thr Leu Val Gly Ile Ile Ser Trp Gly Leu Gly
            340                 345                 350

Cys Gly Gln Lys Asp Val Pro Gly Val Tyr Thr Lys Val Thr Asn Tyr
        355                 360                 365

Leu Asp Trp Ile Arg Asp Asn Met Arg Pro  *
    370                 375